# EUROPEAN PATENT SPECIFICATION

(11) **EP 1 266 011 B1**
(45) Date of publication and mention of the grant of the patent: **26.05.2010**
(21) Application number: 01915099.4
(22) Date of filing: 14.03.2001
(51) Int. Cl.: C12N 15/31, C12N 15/67

(54) **FUNGAL TRANSCRIPTIONAL ACTIVATOR USEFUL IN METHODS FOR PRODUCING POLYPEPTIDES**
PILZ TRANSKRIPTIONSAKTIVATOR ZUR VERWENDUNG IN VERFAHREN ZUR HERSTELLUNG VON POLYPEPTIDEN
ACTIVATEUR TRANSCRIPTIONNEL FONGIQUE, UTILISE DANS DES PROCEDES DE PRODUCTION DE POLYPEPTIDES

(30) Priority: 14.03.2000 DK 200000406
(43) Date of publication of application: 18.12.2002
(73) Proprietor: Novozymes A/S, 2880 Bagsvaerd (DK)
(72) Inventor: HJORT, Carsten, M., DK-2765 Smørum (DK); HONDEL, C., MJJ van den, NL-2804 PZ Gouda (NL); PUNT, P., J., NL-3994 XT Houten (NL); SCHUREN, F., H., J., NL-3906 ZK Veenendaal (NL); CHRISTENSEN, Tove, DK-2800 Lyngby (DK)
(86) International application number: PCT/DK2001/000169
(87) International publication number: WO 2001/068864

(56) References cited:
- WO-A-00/20596
- WO-A-95/35385
- WO-A-97/12045
- DATABASE SWISS-PROT [Online] accession no. P39529 , 1 November 1997 (1997-11-01) B PURNELLE ET AL: "Putative 86.7 kda transcriptional regulatory protein in NUC1-NCE1 intergenic region" XP002901831
- DATABASE SWISS-PROT [Online] accession no P38114, 1 February 1995 (1995-02-01) ENTIAN K.-D ET AL: "Putative 126.9 kda transcriptional regulatory protein in YSW1-RIB7 intergenic region" XP002901832
- K L PETERSEN: "A new transcriptional activator for amylase genes in Aspergillus" MOL GEN GENET, vol. 262, 1999, pages 668-676, XP002901833

### SEQUENCE LISTING

<110> Novo Nordisk A/S
<120>
<130>
<160> 54
<170> PatentIn Ver. 2.1
<210> 1
   <211> 4098
   <212> DNA
   <213> E. coli DSM 12294
<220>
   <221> exon
   <222> (977)..(1204)
<220>
   <221> exon
   <222> (1317)..(1718)
<220>
   <221> exon
   <222> (1777)..(2202)
<220>
   <221> exon
   <222> (2253)..(3116)
<220>
   <221> exon
   <222> (3170)..(3247)
<400> 1
<210> 2
   <211> 666
   <212> PRT
   <213> E.coli DSM 12294
<220>
   <223> Description of Artificial Sequence: MBL1213
<400> 2
   Met Thr Arg Thr Val Asp Glu Ile Lys Tyr Glu Thr Pro Ser Ser Trp
    1               5                  10                  15
   Glu His Lys Ser Leu Asp Val Ala Glu Asp Gly Arg Arg Leu Ala Pro
               20                  25                  30
   His Ser Asp Thr Ala Arg Pro Lys Gly Arg Ile Arg Arg Ser Met Thr
           35                  40                  45
   Ala Cys His Thr Cys Arg Lys Leu Lys Thr Arg Cys Asp Leu Asp Pro
       50                  55                  60
   Arg Gly His Ala Cys Arg Arg Cys Leu Ser Leu Arg Ile Asp Cys Lys
   65                  70                  75                  80
   Leu Pro Glu Thr Thr Asp Arg Phe Gln Asp Ser Ala Ala Met Trp Pro
                   85                  90                  95
   Asp Ala Thr Ser Ala Ile Pro Ser Ile Glu Glu Arg Leu Thr Ser Leu
               100                 105                 110
   Glu Arg Cys Met Arg Glu Met Thr Gly Met Met Arg Gln Met Leu Asp
           115                 120                 125
   His Ser Pro Gly Phe Ala Asn Ala Ser Val Pro His Leu Thr Lys Ser
       130                 135                 140
   Ile Ile Thr Asp Glu Thr Ala Ser Met Glu Gly Ser Pro Ser Ser Pro
   145                 150                 155                 160
   Phe Leu Pro Lys Pro Val Arg Leu Ile Gln Asp Leu Gln Ser Asp Phe
                   165                 170                 175
   Phe Gly Glu Ala Glu Thr Ser Pro Val Asp Ser Pro Leu Ser Ser Asp
               180                 185                 190
   Gly Asn Ala Lys Gly Ala Ile Asp Ser Lys Leu Ser Leu Lys Leu Leu
           195                 200                 205
   Gln Thr Phe Val Asp His Phe Gly Ala Cys Val Ser Ile Tyr Asn Leu
       210                 215                 220
   Ser Asp Ile His Asn Asp Met Lys Ala Pro Asp Ser Leu Leu Tyr Asn
   225                 230                 235                 240
   Thr Ala Cys Leu Leu Ala Ser Arg Tyr Val Pro Gly Ile Pro Thr Ser
                   245                 250                 255
   Thr Val His Ala Ile Tyr Leu Gln Val Arg His Ala Val Val Asn Ile
               260                 265                 270
   Leu Trp Glu Lys Pro Pro Leu Lys Tyr Glu Thr Leu Gln Ala Leu Ala
           275                 280                 285
   Leu Leu Cys Leu Trp Pro Ala Thr Ala Gln Lys Glu Pro Pro Met Asp
       290                 295                 300
   Ser Trp Leu Leu Ser Gly Ile Ser Ile Asn His Ala Ile Ile Ala Leu
   305                 310                 315                 320
   Asp Phe Leu Asn Tyr Ala Pro Ser Glu Val Met Val Asp Asn Glu Thr
                   325                 330                 335
   Ala Ala Gln Leu Arg Leu Trp Asn Thr Tyr Cys Leu Thr Gln Leu His
               340                 345                 350
   Phe Ala Val Gly Asn Ala Arg Pro Phe His Ile Gln Gln Arg Tyr Leu
           355                 360                 365
   Asp His Cys Pro Arg Ile Leu Glu His Pro Ala Ala Thr Leu Glu Asp
       370                 375                 380
   Ala Arg Val Val Ala Glu Ile Gln Leu Tyr Leu Met Thr Leu Arg Leu
   385                 390                 395                 400
   Gln Ser Asn Ser Ser Arg Met Arg Leu Ala Asp Leu Asp Tyr Glu Glu
                   405                 410                 415
   Ile Glu Arg Trp Lys Arg Glu Trp Ala His Leu Phe Cys Lys Lys Pro
               420                 425                 430
   Val Leu Val Ser Arg Gly Leu Pro Leu Thr Arg Ala Thr Ala Gly Glu
           435                 440                 445
   Ser Ser Thr Leu Glu Leu Ser Leu Trp Phe Cys Gln Thr Leu Leu His
       450                 455                 460
   Arg Thr Ala Met Arg Leu Gln Pro Arg Ser Asp Arg Leu Ala Ser Glu
   465                 470                 475                 480
   Val Leu Gln Thr Ser Arg Leu Ile Ile Ser Arg Phe Leu Gln Ile Arg
                   485                 490                 495
   Tyr Ser Thr Ala Leu Ser Leu Val Asp Gln Val Tyr Phe Ile Val Gly
               500                 505                 510
   Tyr Ala Ala Leu Asn Leu Cys Asp Phe Asn Leu Met Asp Pro Leu Ile
           515                 520                 525
   Glu Gln Val Gln Met Phe Leu Leu His Leu Ser Pro Asn Glu Asp His
                   530                 535                 540
   Ile Ala Tyr Arg Phe Ser Cys Met Val Ala Glu Phe Lys Arg Arg Cys
   545                 550                 555                 560
   Gly Ser Ala Glu Cys Asn Asp Pro Ser Ser Thr Val Lys Gly Ser Pro
                   565                 570                 575
   Leu Ser Ser Tyr Gly Asp Ser Arg Lys Met Ser Met Gly Gln Ala Pro
               580                 585                 590
   Phe Met Pro Pro Leu Met Asp Gly Met Ile Glu Gly Tyr Gly Phe Glu
           595                 600                 605
   Gln Leu Met Pro Glu Val Met Pro Ser Ser Phe Pro Asp Gly Ile Leu
       610                 615                 620
   Asn Gly Met Pro Val Thr Gly Leu Ala Ala Tyr Arg Ser Ala Thr Leu
   625                 630                 635                 640
   Ser Ser Asn Thr Arg Asp Asp Asn Leu Gln Arg Trp Phe Ser Ser Cys
                   645                 650                 655
   Pro Trp Ala Glu Leu Lys Pro Arg Thr Pro
               660                 665
<210> 3
   <211> 35
   <212> PRT
   <213> Aspergillus niger
<220>
   <223> Description of Artificial Sequence: PepApr
<400> 3
   Met Thr Ala Cys His Thr Cys Arg Lys Leu Lys Thr Arg Cys Asp Leu
    1               5                  10                  15
   Asp Pro Arg Gly His Ala Cys Arg Arg Cys Leu Ser Leu Arg Ile Asp
               20                  25                  30
   Cys Lys Leu
           35
<210> 4
   <211> 2542
   <212> DNA
   <213> Artificial Sequence
<220>
   <223> Description of Artificial Sequence: PCR Fragment prt13 allele
<400> 4
   gacatggatt ggctggccgc cgtgctgccg tccattagcg tctaccccag gtcaagaacg 60
   gactggacgg acccataacc aatctaacca aagccaattt cgtcaattcc cagctggcga 120
   gcacaatccc attcccaggg ttggccgcca actgttaaaa ggcactatgt gtctctccac 180
   ctgcccgccc ccctcgatgg cctgcgcgta ataactattc tactgctttt tgcctcttac 240
   ttgcctcatt attagtattt tactctactc tccagattgc ctgccagcaa ttggtccaaa 300
   gtggactttg tttgatgaca tgactcgaac cgtggacgag atcaaatacg aaacgccttc 360
   ttcatgggag cacaagagct tggacgttgc cgaggatggc aggcgactag ctccccattc 420
   cgacactgct cgtccgaaag gccgcatacg acgatcgatg actgcctgtc acacatgtcg 480
   gaagcttaaa actagatgtg atctagatcc gcgcggtcat gcgtgccgtc gctgtctatc 540
   tctaaggtca gaggcactac ctacctgcca gttgaagctt tgtccttctg aacgcgacat 600
   gatactagtc gtggaatata actgtcccaa ctttgctgac agtccacaat atctttagaa 660
   tcgattgtaa gctgcctgaa acgaccgacc gcttccaaga cagtgctgcg atgtggccag 720
   acgccacctc ggcaattccc tccatcgagg agcgcctcac ctccctagaa agatgcatga 780
   gggagatgac gggcatgatg cgacagatgc tagatcactc cccaggtttc gcaaatgcct 840
   cggttccgca tttgaccaaa agcatcatca cggatgaaac cgcctcgatg gagggaagcc 900
   cgtcgtcccc cttcctgcct aagcccgttc gcctcattca ggacctccag tccgacttct 960
   tcggagaagc agagacttcc cccgttgact cccctctctc cagcgatggt aacgccaagg 1020
   gcgctatcga ctctaagcta tccctcaaat tgttgcaaac gtatgggtat acctgattga 1080
   caattaccaa aaagctgcta atccttggcg caaatcaggt ttgtcgatca ctttggcgct 1140
   tgcgtttcca tttacaatct ctccgacatc cacaacgaca tgaaagcccc cgactcttta 1200
   ctgtataata ctgcatgcct tctagcttca cgctatgtac cggggatacc gacatctacc 1260
   gtgcatgcta tataccttca agtgcgacat gcagtagtca atattttgtg ggaaaaacca 1320
   cccctgaagt atgagaccct ccaagcactt gcacttctct gtctctggcc agcaaccgcc 1380
   cagaaagagc cacccatgga cagctggctg ctgagtggta tctcaattaa ccatgcaatt 1440
   atcgcgctcg atttcctaaa ctatgcgccc tcggaagtca tggtggacaa tgaaacggct 1500
   gcgcagctgc ggctatggaa tacatattgc ttgacacagc tacagtgggt ttcatctaag 1560
   atctcccgtc cagaagatag ctaacaagct ttagttttgc ggtcgggaat gcgcgtcctt 1620
   tccatatcca gcaaagatac cttgaccact gcccacggat actggagcac ccagcagcaa 1680
   ctctggagga cgcaagggtt gtagcagaaa tacagttgta tttgatgaca ttgcggctcc 1740
   agagcaatag cagtcgaatg cggttggcgg accttgacta tgaggaaata gagcgatgga 1800
   agagggagtg ggctcacctt ttctgtaaga agcctgttct tgtttcccgg ggactaccac 1860
   tgacgagagc aacagctggg gaaagttcca cattggagct gagcctttgg ttctgccaga 1920
   cactccttca ccgcacagca atgaggcttc agcccagatc cgacaggctc gcatctgagg 1980
   ttctgcaaac ctcacgtctg ataatatcgc ggttcctcca gatccggtac tctaccgcat 2040
   taagccttgt cgaccaagtc tatttcattg tcggctacgc tgcactgaat ctgtgcgatt 2100
   tcaatcttat ggacccgctt atcgagcaag tgcagatgtt cctgctgcat ctctccccga 2160
   acgaagacca catcgcctac cggttttcgt gcatggtcgc cgagttcaag cggcgatgtg 2220
   gcagtgcgga atgcaatgac ccatcatcca ctgtcaaggg gtctccgtta tcatcctacg 2280
   gcgacagtcg taagatgagc atggggcaag caccgttcat gccaccgctc atggatggca 2340
   tgatcgaggg gtacggcttc gagcaactga tgccagaagt catgccgagt tcctttccgg 2400
   atgggatact caacggaatg cctgtgactg ggctagcagc gtatcggtca gcgacgctgt 2460
   aagtaatcga gatcgggttg gaaaggacat gagtgggggt ggtggtggta gtagcagtaa 2520
   caccagggat gataacctgc ag 2542
<210> 5
   <211> 665
   <212> PRT
   <213> Aspergillus niger
<400> 5
   Met Thr Arg Thr Val Asp Glu Ile Lys Tyr Glu Thr Pro Ser Ser Trp
   1 5 10 15
   Glu His Lys Ser Leu Asp Val Ala Glu Asp Gly Arg Arg Leu Ala Pro
   20 25 30
   His Ser Asp Thr Ala Arg Pro Lys Gly Arg Ile Arg Arg Ser Met Thr
   35 40 45
   Ala Cys His Thr Cys Arg Lys Leu Lys Thr Arg Cys Asp Leu Asp Pro
   50 55 60
   Arg Gly His Ala Cys Arg Arg Cys Leu Ser Leu Arg Ile Asp Cys Lys
   65                  70                  75                  80
   Leu Pro Glu Thr Thr Asp Arg Phe Gln Asp Ser Ala Ala Met Trp Pro
                   85                  90                  95
   Asp Ala Thr Ser Ala Ile Pro Ser Ile Glu Glu Arg Leu Thr Ser Pro
               100                 105                 110
   Glu Arg Cys Met Arg Glu Met Thr Gly Met Met Arg Gln Met Leu Asp
           115                 120                 125
   His Ser Pro Gly Phe Ala Asn Ala Ser Val Pro His Leu Thr Lys Ser
       130                 135                 140
   Ile Ile Thr Asp Glu Thr Ala Ser Met Glu Gly Ser Pro Ser Ser Pro
   145                 150                 155                 160
   Phe Leu Pro Lys Pro Val Arg Leu Ile Gln Asp Leu Gln Ser Asp Phe
                   165                 170                 175
   Phe Gly Glu Ala Glu Thr Ser Pro Val Asp Ser Pro Leu Ser Ser Asp
               180                 185                 190
   Gly Asn Ala Lys Gly Ala Ile Asp Ser Lys Leu Ser Leu Lys Leu Leu
           195                 200                 205
   Gln Thr Phe Val Asp His Phe Gly Ala Cys Val Ser Ile Tyr Asn Leu
       210                 215                 220
   Ser Asp Ile His Asn Asp Met Lys Ala Pro Asp Ser Leu Leu Tyr Asn
   225                 230                 235                 240
   Thr Ala Cys Leu Leu Ala Ser Arg Tyr Val Pro Gly Ile Pro Thr Ser
                   245                 250                 255
   Thr Val His Ala Ile Tyr Leu Gln Val Arg His Ala Val Val Asn Ile
               260                 265                 270
   Leu Trp Glu Lys Pro Pro Leu Lys Tyr Glu Thr Leu Gln Ala Leu Ala
           275                 280                 285
   Leu Leu Cys Leu Trp Pro Ala Thr Ala Gln Lys Glu Pro Pro Met Asp
       290                 295                 300
   Ser Trp Leu Leu Ser Gly Ile Ser Ile Asn His Ala Ile Ile Ala Leu
   305                 310                 315                 320
   Asp Phe Leu Asn Tyr Ala Pro Ser Glu Val Met Val Asp Asn Glu Thr
                   325                 330                 335
   Ala Ala Gln Leu Arg Leu Trp Asn Thr Tyr Cys Leu Thr Gln Leu His
               340                 345                 350
   Phe Ala Val Gly Asn Ala Arg Pro Phe His Ile Gln Gln Arg Tyr Leu
           355                 360                 365
   Asp His Cys Pro Arg Ile Leu Glu His Pro Ala Ala Thr Leu Glu Asp
       370                 375                 380
   Ala Arg Val Val Ala Glu Ile Gln Leu Tyr Leu Met Thr Leu Arg Leu
   385                 390                 395                 400
   Gln Ser Asn Ser Ser Arg Met Arg Leu Ala Asp Leu Asp Tyr Glu Glu
                   405                 410                 415
   Ile Glu Arg Trp Lys Arg Glu Trp Ala His Leu Phe Cys Lys Lys Pro
               420                 425                 430
   Val Leu Val Ser Arg Gly Leu Pro Leu Thr Arg Ala Thr Ala Gly Glu
           435                 440                 445
   Ser Ser Thr Leu Glu Leu Ser Leu Trp Phe Cys Gln Thr Leu Leu His
       450                 455                 460
   Arg Thr Ala Met Arg Leu Gln Pro Arg Ser Asp Arg Leu Ala Ser Glu
   465                 470                 475                 480
   Val Leu Gln Thr Ser Arg Leu Ile Ile Ser Arg Phe Leu Gln Ile Arg
                   485                 490                 495
   Tyr Ser Thr Ala Leu Ser Leu Val Asp Gln Val Tyr Phe Ile Val Gly
               500                 505                 510
   Tyr Ala Ala Leu Asn Leu Cys Asp Phe Asn Leu Met Asp Pro Leu Ile
           515                 520                 525
   Glu Gln Val Gln Met Phe Leu Leu His Leu Ser Pro Asn Glu Asp His
       530                 535                 540
   Ile Ala Tyr Arg Phe Ser Cys Met Val Ala Glu Phe Lys Arg Arg Cys
   545                 550                 555                 560
   Gly Ser Ala Glu Cys Asn Asp Pro Ser Ser Thr Val Lys Gly Ser Pro
                   565                 570                 575
   Leu Ser Ser Tyr Gly Asp Ser Arg Lys Met Ser Met Gly Gln Ala Pro
               580                 585                 590
   Phe Met Pro Pro Leu Met Asp Gly Met Ile Glu Gly Tyr Gly Phe Glu
           595                 600                 605
   Gln Leu Met Pro Glu Val Met Pro Ser Ser Phe Pro Asp Gly Ile Leu
       610                 615                 620
   Asn Gly Met Pro Val Thr Gly Leu Ala Ala Tyr Arg Ser Ala Thr Leu
   625                 630                 635                 640
   Ser Ser Asn Thr Arg Asp Asp Asn Leu Gln Arg Trp Phe Ser Ser Cys
                   645                 650                 655
   Pro Trp Ala Glu Leu Lys Pro Arg Thr
               660                 665
<210> 6
   <211> 29
   <212> DNA
   <213> Artificial Sequence
<220>
   <223> Description of Artificial Sequence: PepApr
<400> 6
   cggaattcgc atgctggagg tgcttctaa 29
<210> 7
   <211> 33
   <212> DNA
   <213> Artificial Sequence
<220>
   <223> Description of Artificial Sequence: PepA/amdS
<400> 7
   ttcccaggat tgaggcattt tgaccacgag att 33
<210> 8
   <211> 17
   <212> DNA
   <213> Artificial Sequence
<220>
   <223> Description of Artificial Sequence: MBL1213
<400> 8
   taacttccac cgaggtc 17
<210> 9
   <211> 18
   <212> DNA
   <213> Artificial Sequence
<220>
   <223> Description of Artificial Sequence: Primer 122958
<400> 9
   cgatcgatga ctgcctgt 18
<210> 10
   <211> 18
   <212> DNA
   <213> Artificial Sequence
<220>
   <223> Description of Artificial Sequence: Primer 122956
<400> 10
   agagacacat agtgcctt 18
<210> 11
   <211> 18
   <212> DNA
   <213> Artificial Sequence
<220>
   <223> Description of Artificial Sequence: Primer 122959
<400> 11
   gcctatagtc gatagcgc 18
<210> 12
   <211> 18
   <212> DNA
   <213> Artificial Sequence
<220>
   <223> Description of Artificial Sequence: Primer 122960
<400> 12
   cctctctcca gcgatggt 18
<210> 13
   <211> 18
   <212> DNA
   <213> Artificial Sequence
<220>
   <223> Description of Artificial Sequence: Primer122962
<400> 13
   atggaataca tactgctt 18
<210> 14
   <211> 18
   <212> DNA
   <213> Artificial Sequence
<220>
   <223> Description of Artificial Sequence: Primer122961
<400> 14
   atgaaaccca ctgtagct 18
<210> 15
   <211> 18
   <212> DNA
   <213> Artificial Sequence
<220>
   <223> Description of Artificial Sequence: Primer 122963
<400> 15
   tgctcgataa gcgggtcc 18
<210> 16
   <211> 18
   <212> DNA
   <213> Artificial Sequence
<220>
   <223> Description of Artificial Sequence: Primer 122964
<400> 16
   attcttatgg acccgctt 18
<210> 17
   <211> 20
   <212> DNA
   <213> Artificial Sequence
<220>
   <223> Description of Artificial Sequence: Primer 124289
<400> 17
   ccccgggaaa caagaacagg 20
<210> 18
   <211> 20
   <212> DNA
   <213> Artificial Sequence
<220>
   <223> Description of Artificial Sequence: Primer 124290
<400> 18
   gttggcggac cttgactatg 20
<210> 19
   <211> 22
   <212> DNA
   <213> Artificial Sequence
<220>
   <223> Description of Artificial Sequence: Primer 125112
<400> 19
   acagctacag tgggtttcat ct 22
<210> 20
   <211> 19
   <212> DNA
   <213> Artificial Sequence
<220>
   <223> Description of Artificial Sequence:Primer 125111
<400> 20
   agtcaacggg ggaagtctc 19
<210> 21
   <211> 20
   <212> DNA
   <213> Artificial Sequence
<220>
   <223> Description of Artificial Sequence: Primer 128330
<400> 21
   ctagcagcgt atcggtcagc 20
<210> 22
   <211> 19
   <212> DNA
   <213> Artificial Sequence
<220>
   <223> Description of Artificial Sequence: Primer 130887
<400> 22
   cttggaaaag aaacgatag 19
<210> 23
   <211> 19
   <212> DNA
   <213> Artificial Sequence
<220>
   <223> Description of Artificial Sequence: Primer 130888
<400> 23
   aacgtacgct ttcctcctt 19
<210> 24
   <211> 21
   <212> DNA
   <213> Artificial Sequence
<220>
   <223> Description of Artificial Sequence: Primer 134135
<400> 24
   gggtccgtcc agtccgttct t 21
<210> 25
   <211> 24
   <212> DNA
   <213> Artificial Sequence
<220>
   <223> Description of Artificial Sequence: Primer -48 reverse
<400> 25
   agcggataac aatttcacac agga 24
<210> 26
   <211> 17
   <212> DNA
   <213> Artificial Sequence
<220>
   <223> Description of Artificial Sequence: Primer-40 Universal
<400> 26
   gttttcccag tcacgac 17
<210> 27
   <211> 19
   <212> DNA
   <213> Artificial Sequence
<220>
   <223> Description of Artificial Sequence: Primer PstI
<400> 27
   tcatccctgg tgttactgc 19
<210> 28
   <211> 17
   <212> DNA
   <213> Artificial Sequence
<220>
   <223> Description of Artificial Sequence: PstII
<400> 28
   catggattgg ctggccg 17
<210> 29
   <211> 18
   <212> DNA
   <213> Artificial Sequence
<220>
   <223> Description of Artificial Sequence: Prt270n
<400> 29
   tactctccag attgcctg 18
<210> 30
   <211> 18
   <212> DNA
   <213> Artificial Sequence
<220>
   <223> Description of Artificial Sequence: Prt1420r
<400> 30
   tgagatacca ctcagcag 18
<210> 31
   <211> 18
   <212> DNA
   <213> Artificial Sequence
<220>
   <223> Description of Artificial Sequence: Prt1350n
<400> 31
   tgcacttctc tgtctctg 18
<210> 32
   <211> 18
   <212> DNA
   <213> Artificial Sequence
<220>
   <223> Description of Artificial Sequence: Prt2365r
<400> 32
   gacttctggc atcagttg 18
<210> 33
   <211> 18
   <212> DNA
   <213> Artificial Sequence
<220>
   <223> Description of Artificial Sequence: Prt2320n
<400> 33
   ctcatggatg gcatgatc 18
<210> 34
   <211> 33
   <212> PRT
   <213> Aspergillus niger
<220>
   <221> ZN_FING
   <222> (1)..(33)
<400> 34
   Met Thr Ala Cys His Thr Cys Arg Lys Leu Lys Thr Arg Cys Asp Leu
     1               5                  10                  15
   Asp Pro Arg Gly His Ala Cys Arg Arg Cys Leu Ser Leu Arg Ile Asp
                20                  25                  30
   Cys
<210> 35
   <211> 20
   <212> DNA
   <213> Artificial Sequence
<220>
   <223> Description of Artificial Sequence: Degenerated Primer 137396
<400> 35
   atgacygcyt gycayacytg 20
<210> 36
   <211> 20
   <212> DNA
   <213> Artificial Sequence
<220>
   <223> Description of Artificial Sequence: Degenerated Primer 137397
<400> 36
   arrcancgnc grcargcrtg 20
<210> 37
   <211> 20
   <212> DNA
   <213> Artificial Sequence
<220>
   <223> Description of Artificial Sequence: Primer 137394
<400> 37
   atgactgcct gtcacacatg 20
<210> 38
   <211> 20
   <212> DNA
   <213> Artificial Sequence
<220>
   <223> Description of Artificial Sequence: Primer 137395
<400> 38
   agacagcgac ggcacgcatg 20
<210> 39
   <211> 21
   <212> DNA
   <213> Artificial Sequence
<220>
   <223> Description of Artificial Sequence: Primer 144428
<400> 39
   caccgagttt taagcttgcg g 21
<210> 40
   <211> 20
   <212> DNA
   <213> Artificial Sequence
<220>
   <223> Description of Artificial Sequence: Primer 144429
<400> 40
   gcgatcttga tccacgaggg 20
<210> 41
   <211> 21
   <212> DNA
   <213> Artificial Sequence
<220>
   <223> Description of Artificial Sequence: Primer 153468
<400> 41
   cgggatgaat tgtagagagg c 21
<210> 42
   <211> 25
   <212> DNA
   <213> Artificial Sequence
<220>
   <223> Description of Artificial Sequence: Primer 140358
<400> 42
   cgcaagctta aaactcggtg cgatc 25
<210> 43
   <211> 20
   <212> DNA
   <213> Artificial Sequence
<220>
   <223> Description of Artificial Sequence: Primer 140359
<400> 43
   cctcgtggat caagatcgca 20
<210> 44
   <211> 22
   <212> DNA
   <213> Artificial Sequence
<220>
   <223> Description of Artificial Sequence: Primer 175653
<400> 44
   gatgaaaaga ataatcggcg ag 22
<210> 45
   <211> 21
   <212> DNA
   <213> Artificial Sequence
<220>
   <223> Description of Artificial Sequence: Primer 175654
<400> 45
   cgcggcacac tacccccgtt g 21
<210> 46
   <211> 22
   <212> DNA
   <213> Artificial Sequence
<220>
   <223> Description of Artificial Sequence: Primer B043G08
<400> 46
   atctagctca agcattagcg gc 22
<210> 47
   <211> 21
   <212> DNA
   <213> Artificial Sequence
<220>
   <223> Description of Artificial Sequence: Primer B0403G09
<400> 47
   aatttcggcc ctttagtgtc c 21
<210> 48
   <211> 2931
   <212> DNA
   <213> Aspergillus oryzae
<220>
   <221> intron
   <222> (1028)..(1135)
<220>
   <221> intron
   <222> (1538)..(1591)
<220>
   <221> intron
   <222> (2018)..(2066)
<220>
   <221> intron
   <222> (2297)..(2347)
<400> 48
   gatatctcat gatctgcgtg atcggcttgc ctcctatctt agatcacccg ggcttcttca 60
   aatcagcaac aacgctcaga catgtcccct gagaggtgat ccaaatcata cacgagagaa 120
   cgcggaaacg caaattaagg atgagcgaaa aagagaaaaa aatccgttgt tcctgagtca 180
   tgacgaatga gcaaaagtca aacacacctt ctgcttttgg ggggtatgcc cgatcacaat 240
   cttcaacccg ccatgataag agacacacgc tatcgacaaa tcaccggagg tcaagattag 300
   tggcagtcct tagctaattt caggtcggcg tcaaccttag ccaacccaac ccaaccccct 360
   catggaagcg ggactcccta tggagccggc ttacatcggg cgcactgcaa tggcgcacgt 420
   caatcaaccc ctctcttgtt gcagtgccta gtatgccaaa ccaccctttc tattcttcta 480
   gaaaccacac cctagagact cggatctaca cggattggtt ggaatgctcc gattagttgg 540
   catttacccc aggtcaaaat ggataatcaa tctaacggag tctatttcgt caactgcctg 600
   ccagctagca caatctcctc ttcacgcccg gccgtgggct gttaaaaggg tcaattccct 660
   ccccacctgt gtggattctc tatgatttgc acgggatctg acttggtttc cacaattctt 720
   cttgctctca gcttgttcta ctcgccgatt attcttttca tcaacgcggc acactacccc 780
   cgttgtctga tgtcatgact agaactactg ttgaacctat caaatatgag gccccttcgt 840
   gggagcataa gagcgtgcat gtgtccgacg accacaggag aatcatcccc aatgtcggcg 900
   acgacgcgac gcgcccaaag ggccgcatta gacgttcaat gaccgcttgt aatacctgcc 960
   gcaagcttaa aactcggtgc gatcttgatc cacgagggca tgcatgccgg cggtgtctat 1020
   ctttaaggtc gggtgccacc gttatccact ttgtcaaatc tcttacgtca aaatggggga 1080
   tcccatgtcc tgccaagacc aaataagcct ttcttgagta ctaatgtttc tataggatcg 1140
   actgtcagct ccccgagacg agtgagcgct ttcaggacag tactccaatg tggtcagacg 1200
   caacgacagc tatcccctcc atcgaggagc gtctcacttc cctagagagg agtatgagag 1260
   agatgaccgg catgcttcgg cagatcttga atcaatcacc aagcgtctct aatatctccg 1320
   tccctccgct agctcggagt gttcatacgg aagaaacggc ctccattgaa ggaaactcat 1380
   tcggtccttt cctacctaaa cccgttcggc taattcagga cctccaatct gagttttttg 1440
   gggagacaaa ccgcatccct gttgaatctc ctttcttggg taacagtttt gagaagggta 1500
   tcttagattc taagttgtct ctcaagttgg tacagctgta tggtcactcg tcatgtccat 1560
   ctgcctctat agccgctaat gcttgagcta gatttgtgga taatttcggc cctttagtgt 1620
   ccataaataa tcagtcggac ttccacaacg agatgaggaa caccgattcg ttgttatata 1680
   gtactgcctg tcttctggcc tcccgatatg tgccaggcat accaccaccg attgtccata 1740
   ccatgaacct ccaagttcga cataaggcag tcaatctgct gtgggaagaa ccgcctttga 1800
   aatacgaatc gctccaggca ctcgcccttc tttgtttatg gccagcggcg ggtcaaaagg 1860
   agttccccat agatggctgg ttactgagcg ggactgcaat caatcatgcc ctcgtctcct 1920
   ttgacttcct caatcatgtg ccttcagagc ttctcattga taacgatatc gccgctcaat 1980
   tgcggctctg gaacgctttc tgtttaacac agttacagta ggtacaacat ttccggctta 2040
   actccaactt gctaatgcag aaatagtttc gctgttggca acgcacgtcc attccattta 2100
   ccacagagat atctcgatta ttgcccacga cttcttgagc accccgctgc aacagttgag 2160
   gatggcaagg tcgtagcaga gatccagttg tacttgatca cattgcgact ccaagccaac 2220
   gagcaacgta tgcgattcgc ggaggttgaa tacgaagaga ttgaacgatg gaaagttgaa 2280
   tgggcccatc ttcttggtaa ggttaagcaa cgaggaccat ctcatataaa tgctaactat 2340
   tcaacagctg gtgatgaaaa ttcaacattt gagcttagtc tctggttctg tcaaatcctc 2400
   ctgcatcgga cagcaatgag gttccaagcg gagtctgaga gactcacgtc ggaaattctc 2460
   caaggatcgc gcttgatcat ctcgaaattc ctgcaactcc gatttgtcac cgctctaaga 2520
   gtggtcgatc aggcgtactt catcgtcggt tatgccgctc taaatctttg cgacttcaac 2580
   ttcctcgacc ccctcattga ccagatccag atgtttctgc tgcatctgtc gccaaacgaa 2640
   gaccacatcg cataccggtt ttcgtgcatg atagccgagt tcaagcgtcg ctgtgccgaa 2700
   tgcaacgacc cttgcagcgc agtcgacggt tctcaatgct cgttcggaga tgcccggaag 2760
   atgagcatgg aacaggtaca attcgtgcca ccactagtag atagcatgat tgggggatat 2820
   agcgctctgg aacagctgat ccctgaggtc atgccacact catttccgga aagtgtcata 2880
   agtggcatgg ctgtgactga agccatccct gtgggatcgg cgccatacta g          2931
<210> 49
   <211> 624
   <212> PRT
   <213> Aspergillus oryzae
<400> 49
   Met Thr Arg Thr Thr Val Glu Pro Ile Lys Tyr Glu Ala Pro Ser Trp
   1               5                   10                  15
   Glu His Lys Ser Val His Val Ser Asp Asp His Gly Arg Ile Ile Pro
               20                  25                  30
   Asn Val Gly Asp Asp Ala Thr Arg Pro Lys Gly Arg Ile Arg Arg Ser
           35                  40                  45
   Met Thr Ala Cys Asn Thr Cys Arg Lys Leu Lys Thr Arg Cys Asp Leu
       50                  55                  60
   Asp Pro Arg Gly His Ala Cys Arg Arg Cys Leu Ser Leu Arg Ile Asp
   65                  70                  75                  80
   Cys Gln Leu Pro Glu Thr Ser Glu Arg Phe Gln Asp Ser Thr Pro Met
                   85                  90                  95
   Trp Ser Asp Ala Thr Thr Ala Ile Pro Ser Ile Glu Glu Arg Leu Thr
               100                 105                 110
   Ser Leu Glu Arg Ser Met Arg Glu Met Thr Gly Met Leu Arg Gln Ile
           115                 120                 125
   Leu Asn Gln Ser Pro Ser Val Ser Asn Ile Ser Val Pro Pro Leu Ala
       130                 135                 140
   Arg Ser Val His Thr Glu Glu Thr Ala Ser Ile Glu Gly Asn Ser Phe
   145                 150                 155                 160
   Gly Pro Phe Leu Pro Lys Pro Val Arg Leu Ile Gln Asp Leu Gln Ser
                   165                 170                 175
   Glu Phe Phe Gly Glu Thr Asn Arg Ile Pro Val Glu Ser Pro Phe Leu
               180                 185                 190
   Gly Asn Ser Phe Glu Lys Gly Ile Leu Asp Ser Lys Leu Ser Leu Lys
           195                 200                 205
   Leu Val Gln Leu Phe Val Asp Asn Phe Gly Pro Leu Val Ser Ile Asn
       210                 215                 220
   Asn Gln Ser Asp Phe His Asn Glu Met Arg Asn Thr Asp Ser Leu Leu
   225                 230                 235                 240
   Tyr Ser Thr Ala Cys Leu Leu Ala Ser Arg Tyr Val Pro Gly Ile Pro
                   245                 250                 255
   Pro Pro Ile Val His Thr Met Asn Leu Gln Val Arg His Lys Ala Val
               260                 265                 270
   Asn Leu Leu Trp Glu Glu Pro Pro Leu Lys Tyr Glu Ser Leu Gln Ala
           275                 280                 285
   Leu Ala Leu Leu Cys Leu Trp Pro Ala Ala Gly Gln Lys Glu Phe Pro
       290                 295                 300
   Ile Asp Gly Trp Leu Leu Ser Gly Thr Ala Ile Asn His Ala Leu Val
   305                 310                 315                 320
   Ser Phe Asp Phe Leu Asn His Val Pro Ser Glu Leu Leu Ile Asp Asn
                   325                 330                 335
   Asp Ile Ala Ala Gln Leu Arg Leu Trp Asn Ala Phe Cys Leu Thr Gln
               340                 345                 350
   Leu His Phe Ala Val Gly Asn Ala Arg Pro Phe His Leu Pro Gln Arg
           355                 360                 365
   Tyr Leu Asp Tyr Cys Pro Arg Leu Leu Glu His Pro Ala Ala Thr Val
       370                 375                 380
   Glu Asp Gly Lys Val Val Ala Glu Ile Gln Leu Tyr Leu Ile Thr Leu
   385                 390                 395                 400
   Arg Leu Gln Ala Asn Glu Gln Arg Met Arg Phe Ala Glu Val Glu Tyr
                   405                 410                 415
   Glu Glu Ile Glu Arg Trp Lys Val Glu Trp Ala His Leu Leu Ala Gly
               420                 425                 430
   Asp Glu Asn Ser Thr Phe Glu Leu Ser Leu Trp Phe Cys Gln Ile Leu
           435                 440                 445
   Leu His Arg Thr Ala Met Arg Phe Gln Ala Glu Ser Glu Arg Leu Thr
       450                 455                 460
   Ser Glu Ile Leu Gln Gly Ser Arg Leu Ile Ile Ser Lys Phe Leu Gln
   465                 470                 475                 480
   Leu Arg Phe Val Thr Ala Leu Arg Val Val Asp Gln Ala Tyr Phe Ile
                   485                 490                 495
   Val Gly Tyr Ala Ala Leu Asn Leu Cys Asp Phe Asn Phe Leu Asp Pro
               500                 505                 510
   Leu Ile Asp Gln Ile Gln Met Phe Leu Leu His Leu Ser Pro Asn Glu
           515                 520                 525
   Asp His Ile Ala Tyr Arg Phe Ser Cys Met Ile Ala Glu Phe Lys Arg
       530                 535                 540
   Arg Cys Ala Glu Cys Asn Asp Pro Cys Ser Ala Val Asp Gly Ser Gln
   545                 550                 555                 560
   Cys Ser Phe Gly Asp Ala Arg Lys Met Ser Met Glu Gln Val Gln Phe
                   565                 570                 575
   Val Pro Pro Leu Val Asp Ser Met Ile Gly Gly Tyr Ser Ala Leu Glu
               580                 585                 590
   Gln Leu Ile Pro Glu Val Met Pro His Ser Phe Pro Glu Ser Val Ile
           595                 600                 605
   Ser Gly Met Ala Val Thr Glu Ala Ile Pro Val Gly Ser Ala Pro Tyr
       610                 615                 620
<210> 50
   <211> 6015
   <212> DNA
   <213> Artificial Sequence
<220>
   <223> Description of Artificial Sequence: pDV8 plasmid
<400> 50
   gaatacacgg aattcctcga gtaccattta attctatttg tgtttgatcg agacctaata 60
   cagcccctac aacgaccatc aaagtcgtat agctaccagt gaggaagtgg actcaaatcg 120
   acttcagcaa catctcctgg ataaacttta agcctaaact atacagaata agataggtgg 180
   agagcttata ccgagctccc aaatctgtcc agatcatggt tgaccggtgc ctggatcttc 240
   ctatagaatc atccttattc gttgacctag ctgattctgg agtgacccag agggtcatga 300
   cttgagccta aaatccgccg cctccaccat ttgtagaaaa atgtgacgaa ctcgtgagct 360
   ctgtacagtg accggtgact ctttctggca tgcggagaga cggacggacg cagagagaag 420
   ggctgagtaa taagccactg gccagacagc tctggcggct ctgaggtgca gtggatgatt 480
   attaatccgg gaccggccgc ccctccgccc cgaagtggaa aggctggtgt gcccctcgtt 540
   gaccaagaat ctattgcatc atcggagaat atggagcttc atcgaatcac cggcagtaag 600
   cgaaggagaa tgtgaagcca ggggtgtata gccgtcggcg aaatagcatg ccattaacct 660
   aggtacagaa gtccaattgc ttccgatctg gtaaaagatt cacgagatag taccttctcc 720
   gaagtaggta gagcgagtac ccggcgcgta agctccctaa ttggcccatc cggcatctgt 780
   agggcgtcca aatatcgtgc ctctcctgct ttgcccggtg tatgaaaccg gaaaggccgc 840
   tcaggagctg gccagcggcg cagaccggga acacaagctg gcagtcgacc catccggtgc 900
   tctgcactcg acctgctgag gtccctcagt ccctggtagg cagctttgcc ccgtctgtcc 960
   gcccggtgtg tcggcggggt tgacaaggtc gttgcgtcag tccaacattt gttgccatat 1020
   tttcctgctc tccccaccag ctgtagatct tggtggcgtg aaactcccgc acctcttcgg 1080
   ccagcgcctt gtagaagcgc gtatggcttc gtaccccggc catcaacacg cgtctgcgtt 1140
   cgaccaggct gcgcgttctc gcggccatag caaccgacgt acggcgttgc gccctcgccg 1200
   gcagcaagaa gccacggaag tccgcccgga gcagaaaatg cccacgctac tgcgggttta 1260
   tatagacggt ccccacggga tggggaaaac caccaccacg caactgctgg tggccctggg 1320
   ttcgcgcgac gatatcgtct acgtacccga gccgatgact tactggcggg tgctgggggc 1380
   ttccgagaca atcgcgaaca tctacaccac acaacaccgc ctcgaccagg gtgagatatc 1440
   ggccggggac gcggcggtgg taatgacaag cgcccagata acaatgggca tgccttatgc 1500
   cgtgaccgac gccgttctgg ctcctcatat cgggggggag gctgggagct cacatgcccc 1560
   gcccccggcc ctcaccctca tcttcgaccg ccatcccatc gccgccctcc tgtgctaccc 1620
   ggccgcgcgg taccttatgg gcagcatgac cccccaggcc gtgctggcgt tcgtggccct 1680
   catcccgccg accttgcccg gcaccaacat cgtgcttggg gcccttccgg aggacagaca 1740
   catcgaccgc ctggccaaac gccagcgccc cggcgagcgg ctggacctgg ctatgctggc 1800
   tgcgattcgc cgcgtttacg ggctacttgc caatacggtg cggtatctgc agtgcggcgg 1860
   gtcgtggcgg gaggactggg gacagctttc ggggacggcc gtgccgcccc agggtgccga 1920
   gccccagagc aacgcgggcc cacgacccca tatcggggac acgttattta ccctgtttcg 1980
   gggccccgag ttgctggccc ccaacggcga cctgtataac gtgtttgcct gggccttgga 2040
   cgtcttggcc aaacgcctcc gttccatgca cgtctttatc ctggattacg accaatcgcc 2100
   cgccggctgc cgggacgccc tgctgcaact tacctccggg atggtccaga cccacgtcac 2160
   cacccccggc tccataccga cgatatgcga cctggcgcgc acgtttgccc gggagatggg 2220
   ggaggctaac tgaaacacgg aaggagacaa taccggaagg aacccgcgct atccggatcc 2280
   acttaacgtt actgaaatca tcaaacagct tgacgaatct ggatataaga tcgttggtgt 2340
   cgatgtcagc tccggagttg agacaaatgg tgttcaggat ctcgataaga tacgttcatt 2400
   tgtccaagca gcaaagagtg ccttctagtg atttaatagc tccatgtcaa caagaataaa 2460
   acgcgttttc gggtttacct cttccagata cagctcatct gcaatgcatt aatgcattga 2520
   ctgcaaccta gtaacgcctt caggctccgg cgaagagaag aatagcttag cagagctatt 2580
   ttcattttcg ggagacgaga tcaagcagat caacggtcgt caagagacct acgagactga 2640
   ggaatccgct cttggctcca cgcgactata tatttgtctc taattgtact ttgacatgct 2700
   cctcttcttt actctgatag cttgactatg aaaattccgt caccagccct gggttcgcaa 2760
   agataattgc atgtttcttc cttgaactct caagcctaca ggacacacat tcatcgtagg 2820
   tataaacctc gaaatcattc ctactaagat ggtatacaat agtaaccatg catggttgcc 2880
   tagtgaatgc tccgtaacac ccaatacgcc ggccgaaact tttttacaac tctcctatga 2940
   gtcgtttacc cagaatgcac aggtacactt gtttagaggt aatccttctt tctagaagtc 3000
   ctcgtgtact gtgtaagcgc ccactccaca tctccactcg acctgcaggc atgcaagctt 3060
   ggcgtaatca tggtcatagc tgtttcctgt gtgaaattgt tatccgctca caattccaca 3120
   caacatacga gccggaagca taaagtgtaa agcctggggt gcctaatgag tgagctaact 3180
   cacattaatt gcgttgcgct cactgcccgc tttccagtcg ggaaacctgt cgtgccagag 3240
   cggccgctct gcattaatga atcggccaac gcgcggggag aggcggtttg cgtattgggc 3300
   gctcttccgc ttcctcgctc actgactcgc tgcgctcggt cgttcggctg cggcgagcgg 3360
   tatcagctca ctcaaaggcg gtaatacggt tatccacaga atcaggggat aacgcaggaa 3420
   agaacatgtg agcaaaaggc cagcaaaagg ccaggaaccg taaaaaggcc gcgttgctgg 3480
   cgtttttcca taggctccgc ccccctgacg agcatcacaa aaatcgacgc tcaagtcaga 3540
   ggtggcgaaa cccgacagga ctataaagat accaggcgtt tccccctgga agctccctcg 3600
   tgcgctctcc tgttccgacc ctgccgctta ccggatacct gtccgccttt ctcccttcgg 3660
   gaagcgtggc gctttctcat agctcacgct gtaggtatct cagttcggtg taggtcgttc 3720
   gctccaagct gggctgtgtg cacgaacccc ccgttcagcc cgaccgctgc gccttatccg 3780
   gtaactatcg tcttgagtcc aacccggtaa gacacgactt atcgccactg gcagcagcca 3840
   ctggtaacag gattagcaga gcgaggtatg taggcggtgc tacagagttc ttgaagtggt 3900
   ggcctaacta cggctacact agaaggacag tatttggtat ctgcgctctg ctgaagccag 3960
   ttaccttcgg aaaaagagtt ggtagctctt gatccggcaa acaaaccacc gctggtagcg 4020
   gtggtttttt tgtttgcaag cagcagatta cgcgcagaaa aaaaggatct caagaagatc 4080
   ctttgatctt ttctacgggg tctgacgctc agtggaacga aaactcacgt taagggattt 4140
   tggtcatgag attatcaaaa aggatcttca cctagatcct tttaaattaa aaatgaagtt 4200
   ttaaatcaat ctaaagtata tatgagtaaa cttggtctga cagttaccaa tgcttaatca 4260
   gtgaggcacc tatctcagcg atctgtctat ttcgttcatc catagttgcc tgactccccg 4320
   tcgtgtagat aactacgata cgggagggct taccatctgg ccccagtgct gcaatgatac 4380
   cgcgagaccc acgctcaccg gctccagatt tatcagcaat aaaccagcca gccggaaggg 4440
   ccgagcgcag aagtggtcct gcaactttat ccgcctccat ccagtctatt aattgttgcc 4500
   gggaagctag agtaagtagt tcgccagtta atagtttgcg caacgttgtt gccattgcta 4560
   caggcatcgt ggtgtcacgc tcgtcgtttg gtatggcttc attcagctcc ggttcccaac 4620
   gatcaaggcg agttacatga tcccccatgt tgtgcaaaaa agcggttagc tccttcggtc 4680
   ctccgatcgt tgtcagaagt aagttggccg cagtgttatc actcatggtt atggcagcac 4740
   tgcataattc tcttactgtc atgccatccg taagatgctt ttctgtgact ggtgagtact 4800
   caaccaagtc attctgagaa tagtgtatgc ggcgaccgag ttgctcttgc ccggcgtcaa 4860
   tacgggataa taccgcgcca catagcagaa ctttaaaagt gctcatcatt ggaaaacgtt 4920
   cttcggggcg aaaactctca aggatcttac cgctgttgag atccagttcg atgtaaccca 4980
   ctcgtgcacc caactgatct tcagcatctt ttactttcac cagcgtttct gggtgagcaa 5040
   aaacaggaag gcaaaatgcc gcaaaaaagg gaataagggc gacacggaaa tgttgaatac 5100
   tcatactctt cctttttcaa tattattgaa gcatttatca gggttattgt ctcatgagcg 5160
   gatacatatt tgaatgtatt tagaaaaata aacaaatagg ggttccgcgc acatttcccc 5220
   gaaaagtgcc acctgacgtc taagaaacca ttattatcat gacattaacc tataaaaata 5280
   ggcgtatcac gaggcccttt cgtctcgcgc gtttcggtga tgacggtgaa aacctctgac 5340
   acatgcagct cccggagacg gtcacagctt gtctgtaagc ggatgccggg agcagacaag 5400
   cccgtcaggg cgcgtcagcg ggtgttggcg ggtgtcgggg ctggcttaac tatgcggcat 5460
   cagagcagat tgtactgaga gtgcaccata tcgacgctct cccttatgcg actcctgcat 5520
   taggaagcag cccagtagta ggttgaggcc gttgagcacc gccgccgcaa ggaatggtgc 5580
   atgcaaggag atggcgccca acagtccccc ggccacgggg cctgccacca tacccacgcc 5640
   gaaacaagcg ctcatgagcc cgaagtggcg agcccgatct tccccatcgg tgatgtcggc 5700
   gatataggcg ccagcaaccg cacctgtggc gccggtgatg ccggccacga tgcgtccggc 5760
   gtagaggatc tggctagcga tgaccctgct gattggttcg ctgaccattt ccggggtgcg 5820
   gaacggcgtt accagaaact cagaaggttc gtccaaccaa accgactctg acggcagttt 5880
   acgagagaga tgatagggtc tgcttcagta agccagatgc tacacaatta ggcttgtaca 5940
   tattgtcgtt agaacgcggc tacaattaat acataacctt atgtatcata cacatacgat 6000
   ttaggtgaca ctata                                                  6015
<210> 51
   <211> 18
   <212> DNA
   <213> Artificial Sequence
<220>
   <223> Description of Artificial Sequence: Primer B1042E05
<400> 51
   cgcgcgtatc ctattgcc 18
<210> 52
   <211> 20
   <212> DNA
   <213> Artificial Sequence
<400> 52
   gccggaaatg ttgtacctac 20
<210> 53
   <211> 80
   <212> DNA
   <213> Artificial Sequence
<220>
   <223> Description of Artificial Sequence: ICA217
<400> 53
   atgaccgctt gtcatacctg ccgcaagctt aaaactcggt gcgatcttga tccacgaggg 60
   catgcctgcc gccgctgcct                                             80
<210> 54
   <211> 80
   <212> DNA
   <213> Artificial Sequence
<220>
   <223> Description of Artificial Sequence: ICA218
<400> 54
   atgactgctt gccacacctg ccgcaagctt aaaactcggt gcgatcttga tccacgaggg 60
   catgcctgcc gccgctgcct                                             80

## Description

### Background of the Invention

### Field of the Invention

The present invention relates to an isolated nucleic acid sequence encoding a polypeptide having transcriptional activation activity and to the polypeptide. The invention also relates to nucleic acid constructs, vectors and host cells comprising the nucleic acid sequence. The invention further relates to host cells useful for the production of polypeptides in which the production or function of the transcriptional activator has been altered, as well as to methods for producing the polypeptides.

### Description of the Related Art

The use of recombinant host cells in the expression of heterologous proteins has in recent years greatly simplified the production of large quantities of commercially valuable proteins which otherwise are obtainable only by purification from their native sources. Currently, there is a varied selection of expression systems from which to choose for the production of any given protein, including eubacterial and eukaryotic hosts. The selection of an appropriate expression system often depends not only on the ability of the host cell to produce adequate yields of the protein in an active state, but, to a large extent, may also be governed by the intended end use of the protein.

One problem frequently encountered is the high level of proteolytic enzymes produced by a given host cell or present in the culture medium. One suggestion has been to provide host organisms deprived of the ability to produce specific proteolytic compounds. For example, WO 90/00192 (Genencor, Inc.) describes filamentous fungal hosts incapable of secreting enzymatically active aspartic proteinase. EP 574 347 (Ciba Geigy AG) describes *Aspergillus* hosts defective in a serine protease of the subtilisin-type. WO 98/12300 (Novo Nordisk A/S) describes hosts defective in a metalloprotease and an alkaline protease. WO 97/12045 (Genencor, Inc.) describes yeast and bacterial host systems, which are rendered protease deficient resulting from a disruption of a promoter sequence involved in the regulation of a protease gene. Further prior art documents consulted in the context of the present invention are: Database Swiss-Prot, Acc. No.: P39529; Database Swiss-Prot, Acc. No.: P38114; WO 95/35385 and WO 00/20596.

Mattern, I.E., et al., (1992. Mol Gen Genet 234:332-336) describe a mutant strain of *Aspergillus niger*, which was shown to have only 1 to 2% of the extracellular protease activity of the parent strain, apparently due to a deficiency of at least two proteases, aspergillopepsin A and aspergillopepsin B. It was suggested that the protease deficient phenotype could result from a regulatory mutation affecting the expression of the genes coding for both proteases.

The initiation of eukaryotic transcription at a specific promoter or set of promoters requires a eukaryotic transcriptional activator which is a polypeptide, but which is not itself part of RNA polymerase. Many transcriptional activators bind to a specific site on the promoter to form a functional promoter necessary for the initiation of transcription of the polypeptide encoding sequence. However, a transcriptional activator may also be incorporated into an initiation complex only in the presence of other polypeptides. Polypeptides with transcriptional activation activity have been described in fungi, and a list of such polypeptides has been published (Dhawale, S.S., and Lane, A.C. 1993. Nucleic Acid Research 21:5537-5546).

### Solution proposed by the invention:

It is an object of the present invention to provide improved methods for increasing production of polypeptides in host cells in which the activity of a transcriptional activator involved in the regulation of protease production has been modified.

### Summary of the Invention

A first aspect of the present invention relates to a nucleic acid sequence, which comprises the DNA sequence shown in SEQ ID NO: 48,and encoding the polypeptide shown in SEQ ID NO: 49, which polypeptide has transcriptional activation activity.

The nucleic acid sequence shown in SEQ ID NO: 48 is the *Aspergillus oryzae* IF04177 *prtT* gene encoding the transcriptional factor shown in SEQ ID NO: 49. The *A. oryzae prtT* gene has a coding region starting in position 795 and ending at position 2931. The *prtT* gene has 4 introns in positions 1028-1135, 1538-1591, 2018-2066, and 2297-2347, respectively. This is described further below and in the Examples.

In another aspect, the invention also relates to a nucleic acid construct, vector and host cells comprising the nucleic acid sequence, and to the polypeptide encoded by the nucleic acid sequence. The invention further relates to host cells useful for the production of a polypeptide, in which the production or function of the transcriptional activator has been altered, as well as to methods for producing the polypeptide.

### Brief Description of the Figures

Figure 1 shows a restriction map of the plasmid pPAP, the construction of which is described in Example 1.
Figure 2 shows a restriction map of the plasmid pAopyrGcosArp1, the construction of which is described in Example 1.
Figure 3 shows a restriction map of the plasmid pEES1, the construction of which is described in Example 1.
Figure 4 shows a restriction map of the plasmid pDprt, the construction of which is described in Example 3.
Figure 5 shows a restriction map of the plasmid pGPprt, the construction of which is described in Example 4.

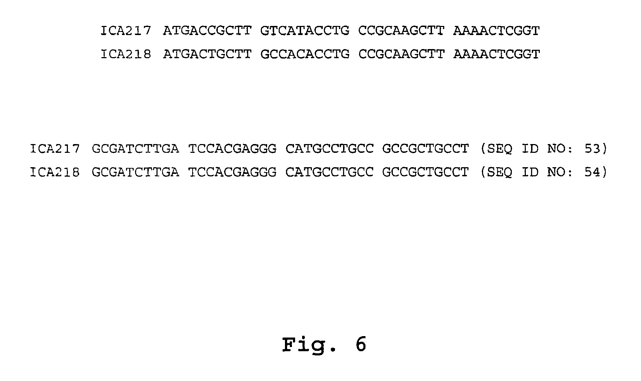

Figure 6 shows the sequence of the insert in the two plasmids containing the PCR fragment of the *A. oryzae prtT* Zn²⁺-finger. ICA217 is the sequence from one of the plasmids and ICA218 is the sequence from the other.
Figure 7 shows plasmid pDV8 a pSP65 (Promega™) based plasmid containing the HSV-tk gene on a 1.2 kb *BglII*/*BamHI* fragment inserted between a 1.0 kb *XhoI*/*BglII* fragment of the *A. nidulans gpd* promoter and a 0.8 kb *BamHI*/*HindIII* fragment containing the *A. nidulans trpC* transcriptional terminator.
Figure 8 shows the construction of pJaL554 described in Example 8.

### Detailed Description of the Invention

### Nucleic Acid Sequences Encoding Transcriptional Activators

A first aspect of the present invention relates to a nucleic acid sequence, which comprises the DNA sequence shown in SEQ ID NO: 48, and encoding the polypeptide shown in SEQ ID NO: 49, which polypeptide has transcriptional activation activity.

The term "transcriptional activator" as used herein refers to a polypeptide which has the capability to activate a specific promoter or set of promoters necessary for the initiation of transcription of the polypeptide encoding sequence to which it is linked.

The term "isolated nucleic acid sequence" as used herein refers to a nucleic acid sequence which is essentially free of other nucleic acid sequences, *e*.*g*., at least about 20% pure, preferably at least about 40% pure, more preferably at least about 60% pure, even more preferably at least about 80% pure, most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

In a preferred embodiment, the nucleic acid sequence encoding a transcriptional activator has a nucleic acid sequence as set forth in SEQ ID NO:48.

Modification of a nucleic acid sequence encoding a polypeptide of the present invention may be necessary for the synthesis of polypeptides substantially similar to the polypeptide. The term "substantially similar" to the polypeptide refers to non-naturally occurring forms of the polypeptide. These polypeptides may differ in some engineered way from the polypeptide isolated from its native source. For example, it may be of interest to synthesize variants of the polypeptide where the variants differ in specific activity, binding specificity and/or affinity, or the like using, *e*.*g*., site-directed mutagenesis. The analogous sequence may be constructed on the basis of the nucleic acid sequence presented as the polypeptide encoding part of SEQ ID NO: 48, *e*.*g*., a subsequence thereof, and/or by introduction of nucleotide substitutions which do not give rise to another amino acid sequence of the polypeptide encoded by the nucleic acid sequence, but which corresponds to the codon usage of the host organism intended for production of the enzyme, or by introduction of nucleotide substitutions which may give rise to a different amino acid sequence. For a general description of nucleotide substitution, see, *e*.*g.,* Ford et al., 1991, Protein Expression and Purification 2: 95-107.

"Fungi" as used herein includes the phyla Ascomycota, Basidiomycota, Chytridiomycota, and Zygomycota (as defined by Hawksworth, et al., in Ainsworth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra*).

In preferred embodiment, the fungal source is a filamentous fungal strain. "Filamentous fungi" include all filamentous forms of the subdivision Eumycota and Oomycota (as defined by Hawksworth *et al*., 1995, *supra*). The filamentous fungi are characterized by a mycelia wall composed of chitin, cellulose, glucan, chitosan, mannan, and other complex polysaccharides. Vegetative growth is by hyphal elongation and carbon catabolism is obligately aerobic. Filamentous fungal strains include, but are not limited to, strains of *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium*, and *Trichoderma.*

The nucleic acid sequence encoding a transcriptional activator of the present invention is obtained from a strain of *Aspergillus oryzae.* Preferably, the nucleic acid sequence is obtained from an isolate of a strain of *A. oryzae* IFO 4177, i.e., the nucleic acid sequence set forth in SEQ ID NO: 48.

In another preferred embodiment, the isolated nucleic acid sequence encodes a polypeptide comprising the amino acid sequence of SEQ ID NO: 49.

### Nucleic Acid Constructs

Another aspect of the present invention relates to nucleic acid constructs comprising a nucleic acid sequence encoding a transcriptional activator of the present invention operably linked to one or more control sequences, which direct the production of the transcriptional activator in a suitable expression host. In a preferred embodiment, the nucleic acid sequence is SEQ ID NO: 48 encoding the polypeptide shown in SEQ ID NO: 49.

Expression will be understood to include any step involved in the production of the polypeptide including, but not limited to, transcription, post-transcriptional modification, translation, post-translational modification, and secretion. Manipulation of the nucleic acid sequence encoding a polypeptide prior to its insertion into a vector may be desirable or necessary depending on the expression vector. The techniques for modifying nucleic acid sequences utilizing cloning methods are well known in the art.

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette when the nucleic acid construct contains all the control sequences required for expression of a coding sequence. The term "coding sequence" as defined herein is a sequence, which is transcribed into mRNA and translated into a transcriptional activator of the present invention. The boundaries of the coding sequence are generally determined by the ATG start codon at the 5' end of the mRNA and a transcription terminator sequence located just downstream of the open reading frame at the 3' end of the mRNA. A coding sequence can include, but is not limited to, DNA, cDNA, and recombinant nucleic acid sequences.

The term "control sequences" is defined herein to include all components, which are necessary or advantageous for the expression of a polypeptide. Each control sequence may be native or foreign to the nucleic acid sequence encoding the polypeptide. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the production of a polypeptide.

The control sequence may be an appropriate promoter sequence, a nucleic acid sequence, which is recognized by a host cell for expression of the nucleic acid sequence. The promoter sequence contains transcriptional control sequences, which mediate the expression of the polypeptide. The promoter may be any nucleic acid sequence, which shows transcriptional activity in the cell including mutant, truncated, and hybrid promoters, and may be obtained from genes encoding extracellular or intracellular polypeptides either homologous or heterologous to the cell.

The control sequence may also be a suitable transcription terminator sequence, a sequence recognized by a filamentous fungal cell to terminate transcription. The terminator sequence is operably linked to the 3' terminus of the nucleic acid sequence encoding the polypeptide. Any terminator, which is functional in the cell, may be used in the present invention.

Preferred terminators for filamentous fungal cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of a mRNA which is important for translation by the filamentous fungal cell. The leader sequence is operably linked to the 5' terminus of the nucleic acid sequence encoding the polypeptide. Any leader sequence, which is functional in the cell, may be used in the present invention.

Preferred leaders for filamentous fungal cells are obtained from the genes encoding *Aspergillus oryzae* TAKA amylase and *Aspergillus nidulans* triose phosphate isomerase.

The control sequence may also be a polyadenylation sequence, a sequence which is operably linked to the 3' terminus of the nucleic acid sequence and which, when transcribed, is recognized by the filamentous fungal cell as a signal to add polyadenosine residues to transcribed mRNA. Any polyadenylation sequence, which is functional in the cell, may be used in the present invention.

Preferred polyadenylation sequences for filamentous fungal cells are obtained from the genes encoding *Aspergillus oryzae* TAKA amylase, *Aspergillus niger* glucoamylase, *Aspergillus nidulans* anthranilate synthase, and *Aspergillus niger* alpha-glucosidase.

The control sequence may also be a signal peptide-coding region, which codes for an amino acid sequence linked to the amino terminus of the polypeptide, which can direct the encoded polypeptide into the cell's secretory pathway. The 5' end of the coding sequence of the nucleic acid sequence may inherently contain a signal peptide-coding region naturally linked in translation reading frame with the segment of the coding region, which encodes the secreted polypeptide. Alternatively, the 5' end of the coding sequence may contain a signal peptide-coding region, which is foreign to the coding sequence. The foreign signal peptide-coding region may be required where the coding sequence does not normally contain a signal peptide-coding region. Alternatively, the foreign signal peptide-coding region may simply replace the natural signal peptide-coding region in order to obtain enhanced secretion of the polypeptide. The signal peptide-coding region may be obtained from a glucoamylase or an amylase gene from an *Aspergillus* species, or a lipase or proteinase gene from a *Rhizomucor* species. However, any signal peptide-coding region, which directs the expressed polypeptide into the secretory pathway of a filamentous fungal cell, may be used in the present invention.

An effective signal peptide coding region for filamentous fungal cells is the signal peptide coding region obtained from the *Aspergillus oryzae* TAKA amylase gene, *Aspergillus niger* neutral amylase gene, *Rhizomucor miehei* aspartic proteinase gene, or *Humicola lanuginosa* cellulase gene.

The control sequence may also be a propeptide coding region, which codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the *Rhizomucor miehei* aspartic proteinase gene, or the *Myceliophthora thermophila* laccase gene (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

### Expression Vectors

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence encoding the polypeptide may be expressed by inserting the sequence or a nucleic acid construct comprising the sequence into an appropriate vector for expression. In creating the expression vector, the coding sequence is located in the vector so that the coding sequence is operably linked with the appropriate control sequences for expression, and possibly secretion.

The recombinant expression vector may be any vector (*e*.*g*., a plasmid or virus), which can be conveniently subjected to recombinant DNA procedures and can bring about the expression of the nucleic acid sequence encoding the polypeptide. The choice of the vector will typically depend on the compatibility of the vector with the filamentous fungal cell into which the vector is to be introduced. The vectors may be linear or closed circular plasmids. The vector may be an autonomously replicating vector, *i*.*e*., a vector, which exists as an extrachromosomal entity, the replication of which is independent of chromosomal replication, *e*.*g*., a plasmid, an extrachromosomal element, a minichromosome, or an artificial chromosome. The vector may contain any means for assuring self-replication. Alternatively, the vector may be one which, when introduced into the filamentous fungal cell, is integrated into the genome and replicated together with the chromosome(s) into which it has been integrated. The vector system may be a single vector or plasmid or two or more vectors or plasmids, which together contain the total DNA to be introduced into the genome of the filamentous fungal cell, or a transposon.

The vectors preferably contain one or more selectable markers, which permit easy selection of transformed cells. A selectable marker is a gene the product of which provides for biocide or viral resistance, resistance to heavy metals, prototrophy to auxotrophs, and the like. A selectable marker for use in a filamentous fungal cell may be selected from the group including, but not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents from other species. Preferred for use in an *Aspergillus* cell are the *amdS* and *pyrG* genes of *Aspergillus nidulans* or *Aspergillus oryzae* and the bar gene of *Streptomyces hygroscopicus*.

The vectors preferably contain an element(s) that permits stable integration of the vector into the host cell genome or autonomous replication of the vector in the cell independent of the genome of the cell.

### Host Cells

Another aspect of the present invention relates to host cells comprising a nucleic acid construct or an expression vector of the present invention.

The choice of a host cell in the methods of the present invention will to a large extent depend upon the source of the nucleic acid sequence encoding the polypeptide of interest.

The introduction of an expression vector or a nucleic acid construct into a filamentous fungal cell may involve a process consisting of protoplast formation, transformation of the protoplasts, and regeneration of the cell wall in a manner known *per se*. Suitable procedures for transformation of *Aspergillus* cells are described in EP 238 023 and Yelton et al., 1984, Proceedings of the National Academy of Sciences USA 81: 1470-1474. A suitable method of transforming *Fusarium* species is described by Malardier et al., 1989, Gene 78: 147-156 or in WO 96/00787.

"Introduction" means introducing a vector comprising the nucleic acid sequence encoding the polypeptide into a filamentous fungal cell so that the vector is maintained as a chromosomal integrant or as a self-replicating extrachromosomal vector. Integration is generally considered to be an advantage as the nucleic acid sequence is more likely to be stably maintained in the cell. Integration of the vector into the chromosome occurs by homologous recombination, nonhomologous recombination, or transposition.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location (s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. These nucleic acid sequences may be any sequence that is homologous with a target sequence in the genome of the host cell, and, furthermore, may be non-encoding or encoding sequences.

For autonomous replication, the vector may further comprise an origin of replication enabling the vector to replicate autonomously in the host cell.

The procedures used to ligate the elements described above to construct the recombinant expression vectors are well known to one skilled in the art (see, *e*.*g*., Sambrook, et al., *supra*).

In a preferred embodiment, the filamentous fungal host cell is a cell of a species of *Aspergillus*.

In a most preferred embodiment, the filamentous fungal cell is an *Aspergillus oryzae* cell.

### Polypeptides having Transcriptional Activation Activity

As defined herein, an "isolated" polypeptide is a polypeptide which is essentially free of other polypeptides, *e*.*g*., at least about 20% pure, preferably at least about 40% pure, more preferably about 60% pure, even more preferably about 80% pure, most preferably about 90% pure, and even most preferably about 95% pure, as determined by SDS-PAGE.

In a preferred embodiment, the transcriptional activator of the present invention comprises the amino acid sequence of SEQ ID NO: 49. In a most preferred embodiment, the polypeptide has the amino acid sequence of SEQ ID NO: 49.

The amino acid sequences of a homologous polypeptides may differ from the amino acid sequence of SEQ ID NO: 49 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (such as arginine, lysine and histidine), acidic amino acids (such as glutamic acid and aspartic acid), polar amino acids (such as glutamine and asparagine), hydrophobic amino acids (such as leucine, isoleucine and valine), aromatic amino acids (such as phenylalanine, tryptophan and tyrosine), and small amino acids (such as glycine, alanine, serine, threonine and methionine). Amino acid substitutions, which do not generally alter the specific activity are known in the art and are described, for example, by H. Neurath and R.L. Hill (1979. The Proteins, Academic Press, New York). The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

In another preferred embodiment, the transcriptional activator of the present invention is the polypeptide encoded by the nucleic acid sequence shown in SEQ ID NO: 48 encoding the polypeptide shown in SEQ ID NO: 49.

The present invention further relates to methods for producing the transcriptional activator of the present invention comprising (a) cultivating a host cell harbouring a nucleic acid construct or an expression vector comprising a nucleic acid sequence encoding the transcriptional activator of the invention under conditions conducive for production of the polypeptide; and (b) recovering the polypeptide.

### Host Cells having Altered Transcriptional Activation Activity

Another aspect relates to a host cell which is a mutant of a parent fungal cell useful for the production of a polypeptide in which the parent cell comprises one or more nucleic acid sequences encoding a protease, the transcription of which is activated by a transcriptional activator of the present invention, and the mutant cell produces less of the transcriptional activator and the protease(s) than the parent cell when cultured under the same conditions.

The mutant cell may be constructed using methods well known in the art; for example, by one or more nucleotide insertions or deletions of the gene encoding the transcriptional activator.

In a preferred embodiment the mutant cell is obtained by modification or inactivation of a nucleic acid sequence present in the cell and necessary for expression of the transcriptional activator.

In another preferred embodiment the reduced expression of the transcriptional activator in the mutant cell is obtained by modification or inactivation of a control sequence required for the expression of the transcriptional activator. The term "control sequence" is defined, *supra,* in the section entitled "Nucleic Acid Constructs." In a more preferred embodiment the control sequence in the mutant cell is a promoter sequence or a functional part thereof, *i*.*e*., a part, which is sufficient for affecting expression of the nucleic acid sequence. Other control sequences for possible modification include, but are not limited to, a leader, a polyadenylation sequence, a propeptide sequence, a signal sequence, and a transcription terminator.

Modification or inactivation of the gene may be performed by subjecting the parent cell to mutagenesis and selecting for mutant cells in which the capability to produce a transcriptional activator has been reduced. The mutagenesis, which may be specific or random, may be performed, for example, by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used, the mutagenesis is typically performed by incubating the parent cell to be mutagenized in the presence of the mutagenizing agent of choice under suitable conditions, and selecting for mutant cells exhibiting reduced expression of the gene.

Modification or inactivation of the gene may be accomplished by introduction, substitution, or removal of one or more nucleotides in the gene's nucleic acid sequence or a regulatory element required for the transcription or translation thereof. For example, nucleotides may be inserted or removed so as to result in the introduction of a stop codon, the removal of the start codon, or a change of the open reading frame. Such modification or inactivation may be accomplished by site-directed mutagenesis or PCR generated mutagenesis in accordance with methods known in the art. Although, in principle, the modification may be performed *in vivo, i.e.,* directly on the fungal cell expressing the gene to be modified, it is preferred that the modification be performed *in vitro* as exemplified below.

An example of a convenient way to inactivate or reduce expression of the gene by a fungal cell of choice is based on techniques of gene replacement or gene interruption. For example, in the gene interruption method, a nucleic acid sequence corresponding to the endogenous gene or gene fragment of interest is mutagenized *in vitro* to produce a defective nucleic acid sequence which is then transformed into the parent cell to produce a defective gene. By homologous recombination, the defective nucleic acid sequence replaces the endogenous gene or gene fragment. It may be desirable that the defective gene or gene fragment also encodes a marker, which may be used for selection of transformants in which the nucleic acid sequence has been modified or destroyed.

Alternatively, modification or inactivation of the gene may be performed by established anti-sense techniques using a nucleotide sequence complementary to the nucleic acid sequence of the gene. More specifically, expression of the gene by a filamentous fungal cell may be reduced or eliminated by introducing a nucleotide sequence complementary to the nucleic acid sequence, which may be transcribed in the cell and is capable of hybridizing to the mRNA produced in the cell. Under conditions allowing the complementary anti-sense nucleotide sequence to hybridize to the mRNA, the amount of protein translated is thus reduced or eliminated.

A nucleic acid sequence complementary to the nucleic acid sequence of SEQ ID NO: 48 may be obtained from any microbial source. The preferred sources are fungal sources, *e.g.,* yeast and filamentous fungi as described *supra*. Preferred filamentous fungal sources include, but are not limited to, species of *Acremonium, Aspergillus, Fusarium, Humicola, Myceliophthora, Mucor, Neurospora, Penicillium, Phanerochaete, Thielavia, Tolypocladium,* and *Trichoderma.* Preferred yeast sources include, but are not limited to, species of *Candida, Hansenula, Kluyveromyces, Pichia, Saccharomyces, Schizosaccharomyces*, and *Yarrowia*. Furthermore, the nucleic acid sequence may be native to the filamentous fungal cell.

In another preferred embodiment, the parent cell harbours a gene having a nucleic acid sequence encoding a polypeptide with an amino acid sequence which has at least 50% identity with the amino acid sequence of SEQ ID NO: 49.

In another preferred embodiment, the parent cell harbours a gene having a nucleic acid sequence with at least 70% identity with the nucleic acid sequence of SEQ ID NO: 48.

In another preferred embodiment, the mutant cell harbours a nucleic acid sequence, which has been modified or inactivated by any of the methods described above and produces less of a protease or a combination of proteases than the parent cell when cultured under identical conditions. The mutant cell produces preferably at least about 25% less, more preferably at least about 50% less, even more preferably at least about 75% less, and even more preferably at least about 95% less of a protease or a combination of proteases than the parent cell when cultured under identical conditions.

In an even more preferred embodiment, the mutant cell produces essentially undetectable amounts of a protease or combination of proteases than the parent cell when cultured under identical conditions.

The protease(s) may be assayed using known methods. In one such method, an aliquot of a 48 hour culture media is incubated with ³H-labelled sperm whale myoglobin at pH 4.0 and the radioactivity in the TCA-soluble fraction is measured (van Noort, J.M., et al., 1991. Anal. Biochem 198:385-390). Other methods have been described for identifying, e.g., aspartic proteinase A. of *A. niger* (Takahashi, K., 1991. Meth. in Enzymol. 248:146-155), endopeptidases (Morihara, K., 1995. Meth. in Enzymol. 248:242-253), carboxypeptidases (Reminton, J., and Breddam, K., 1994. Meth. in Enzymol. 244:231-248), dipeptidyl peptidase (Ikehara, Y., et al., 244:215-227), and aminopeptidases (Little, G., et al., 1976. Meth. in Enzymol. 45:495-503).

In another preferred embodiment, the mutant cell harbours at least one copy of a nucleic acid sequence encoding a polypeptide of interest.

Another aspect relates to a host cell useful for the production of a polypeptide wherein the host cell is a mutant of a parent fungal cell in which the mutant (a) produces more of the transcriptional activator of the present invention as compared to the parent cell when cultured under the same conditions; and (b) comprises a DNA sequence encoding the polypeptide, the transcription of which is activated by the transcriptional activator.

In a preferred embodiment according to the invention, the host cell produces more of the transcriptional activator than the parent cell when cultured under the same conditions by introducing into the parent cell one or more copies of (i) a nucleic acid sequence encoding a transcriptional activator, (ii) a nucleic acid construct comprising a nucleic acid sequence encoding a transcriptional activator, or (iii) an expression vector as defined above in the section "Expression Vectors".

The nucleic acid construct comprising a nucleic acid sequence encoding a transcriptional activator of the present invention may also comprise one or more nucleic acid sequences which encode one or more factors that are advantageous for directing the expression of the polypeptide, *e*.*g*., a transcriptional activator (*e*.*g*., a trans-acting factor), a chaperone, and a processing protease. Any factor that is functional in the filamentous fungal cell of choice may be used in the present invention. The nucleic acids encoding one or more of these factors are not necessarily in tandem with the nucleic acid sequence encoding the polypeptide.

An activator is a protein, which activates transcription of a nucleic acid sequence encoding a polypeptide (Kudla et al., 1990, EMBO Journal 9: 1355-1364; Jarai and Buxton, 1994, Current Genetics 26: 2238-244; Verdier, 1990, Yeast 6: 271-297). The nucleic acid sequence encoding an activator may be obtained from the genes encoding *Saccharomyces cerevisiae* heme activator protein 1 (*hap1*), *Saccharomyces cerevisiae* galactose metabolizing protein 4 (*gal4*), *Aspergillus nidulans* ammonia regulation protein (*areA*), and *Aspergillus oryzae* alpha-amylase activator (*amyR*). For further examples, see Verdier, 1990, *supra* and MacKenzie et al., 1993, Journal of General Microbiology 139: 2295-2307.

A chaperone is a protein which assists another polypeptide in folding properly (Hartl et al., 1994, TIBS 19: 20-25; Bergeron et al., 1994, TIBS 19: 124-128; Demolder et al., 1994, Journal of Biotechnology 32: 179-189; Craig, 1993, Science 260: 1902-1903; Gething and Sambrook, 1992, Nature 355: 33-45; Puig and Gilbert, 1994, Journal of Biological Chemistry 269: 7764-7771; Wang and Tsou, 1993, The FASEB Journal 7: 1515-11157; Robinson et al., 1994, Bio/Technology 1: 381-384; Jacobs et al., 1993, Molecular Microbiology 8: 957-966). The nucleic acid sequence encoding a chaperone may be obtained from the genes encoding *Aspergillus oryzae* protein disulphide isomerase or *Saccharomyces cerevisiae* calnexin, *Saccharomyces cerevisiae* BiP/GRP78, and *Saccharomyces cerevisiae* Hsp70. For further examples, see Gething and Sambrook, 1992, *supra,* and Hartl *et al.*, 1994, *supra.*

A processing protease is a protease that cleaves a propeptide to generate a mature biochemically active polypeptide (Enderlin and Ogrydziak, 1994, Yeast 10: 67-79; Fuller et al., 1989, Proceedings of the National Academy of Sciences USA 86: 1434-1438; Julius et al., 1984, Cell 37: 1075-1089; Julius et al., 1983, Cell 32: 839-852; U.S. Patent No. 5,702,934). The nucleic acid sequence encoding a processing protease may be obtained from the genes encoding *Saccharomyces cerevisiae* dipeptidylaminopeptidase, *Saccharomyces cerevisiae* Kex2, *Yarrowia lipolytica* dibasic processing endoprotease *(xpr6),* and *Fusarium oxysporum* metalloprotease (p45 gene).

In a more preferred embodiment, the nucleic acid sequence encoding the transcriptional activator is operably linked to a promoter, or a functional part thereof, which is stronger than the corresponding promoter of the parent cell. In an even more preferred embodiment, the promoter, or a functional part thereof, mediates the expression of a gene encoding an extracellular protease, such as the *Aspergillus oryzae* alkaline protease, *A. oryzae* neutral metalloprotease, *A. niger* aspergillopepsin protease, *Fusarium oxysporum* trypsin-like protease or *F. venenatum* trypsin.

A further embodiment relates to a host cell useful for the production of a polypeptide wherein the host cell is a mutant of a parent fungal cell in which the mutant comprises
a) a modification or inactivation of a transcriptional activator of the present invention, or a regulatory sequence thereof, and
b) (i) an inducible promoter operably linked to a nucleic acid sequence encoding a transcriptional activator of the present invention, and (ii) a promoter sequence to which the transcriptional activator can bind, operably linked to a nucleic acid sequence encoding the polypeptide, wherein (i) and (ii) can be introduced simultaneously or sequentially.

The inactive form of the transcriptional activator in (a) above is obtained by inactivation or modification of a nucleic acid sequence present in the cell and necessary for the expression of the native transcriptional activator according to any of the methods as disclosed *supra.* In a preferred embodiment the inactivation or modification is obtained by methods, which include, one or more nucleotide insertions, deletions or substitutions, specific or random mutagenesis, gene replacement or gene interruption, and anti-sense techniques using a nucleotide sequence complementary to the nucleic acid sequence of the transcriptional activator. In another preferred embodiment, the inactive form of the native transcriptional activator is obtained by inactivation or modification of a control sequence required for the expression of the transcriptional activator.

In another preferred embodiment, the nucleic acid sequence encoding the native transcriptional activator has the sequence set forth in SEQ ID NO: 48.

The inducible promoter sequence in (b) above may be any promoter sequence, or a functional part thereof, wherein the transcription initiation activity of the promoter can be induced according to the fermentation conditions. Preferably, the induction is mediated by a carbon or nitrogen catabolite. In a preferred embodiment, the promoter is the *amdS* promoter of *Aspergillus nidulans* or *A. oryzae,* the *niaD* promoter of *A. nidulans, A. oryzae* or *A. niger,* the *niiA* promoter of *Aspergillus* species, the alkaline phosphatase promoter of *Aspergillus* sp., the acid phosphatase promoter of *Aspergillus* sp., or the *alcA* promoter of *A. niger*.

In another preferred embodiment, the host cell further comprises a promoter sequence, wherein the promoter sequence can be activated by the transcriptional activator and is operably linked to the nucleic acid sequence encoding the polypeptide.

The promoter sequence activated by the transcriptional activator of the present invention may be any promoter sequence, or a functional part thereof, selected from the group which includes but is not limited to promoters obtained from the genes encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, the NA2-tpi promoter (a hybrid of the promoters from the genes encoding *Aspergillus niger* neutral alpha-amylase and *Aspergillus oryzae* triose phosphate isomerase), and mutant, truncated, and hybrid promoters thereof. Particularly preferred promoters for use in filamentous fungal cells are a promoter, or a functional part thereof, from a protease gene; *e.g.,* from the *Fusarium oxysporum* trypsin-like protease gene (U.S. Patent No. 4,288,627), *Aspegillus oryzae* alkaline protease gene (*alp*), *Aspergillus niger pacA* gene, *Aspergillus oryzae* alkaline protease gene, *A. oryzae* neutral metalloprotease gene, *A. niger* aspergillopepsin protease gene, or *F. venenatum* trypsin gene.

In another preferred embodiment, the host cell harbours at least one copy of a nucleic acid sequence encoding a polypeptide.

In another preferred embodiment, the host cell, which expresses the transcriptional activator of the present invention produces less of one or more native proteases than the parent cell when cultured under identical conditions. The protease(s) may be assayed using any of the methods described above. In a more preferred embodiment, an aliquot from a 48-hour culture media is incubated with ³H-labelled sperm whale myoglobin at pH 4.0 and the radioactivity in the TCA-soluble fraction is measured (van Noort, J.M., *et al., supra*).

The nucleic acid constructs described herein may be introduced into a parent fungal cell according to any of the methods as described *supra* in the section, "Host Cells" to obtain a host cell useful for the production of a polypeptide. In a preferred embodiment the nucleic acid construct is integrated into the chromosome of the cell. In another preferred embodiment the nucleic acid construct is maintained as a self-replicating extra-chromosomal vector.

It will be understood that the methods of the present invention are not limited to a particular order for obtaining the mutant fungal cell. The modification of the second nucleic acid sequence may be introduced into the parent cell at any step in the construction of the cell for the production of a polypeptide.

### Producing a Polypeptide

Another aspect of the present invention relates to methods of producing a polypeptide in a host cell of the present invention, comprising: (a) cultivating the host cell which harbours a gene encoding the polypeptide in a nutrient medium suitable for production of the polypeptide; and (b) recovering the polypeptide from the nutrient medium of the host cell.

In one embodiment, the host cell is a mutant of a parent fungal cell in which the parent cell comprises one or more nucleic acid sequences encoding a protease, the transcription of which is activated by a transcriptional activator of the present invention, and the mutant cell produces less of the transcriptional activator and the protease(s) than the parent cell when cultured under the same conditions.

In another embodiment, the host cell is a mutant of a parent fungal cell in which the mutant (a) produces more of the transcriptional activator of the present invention as compared to the parent cell when cultured under the same conditions; and (b) comprises a DNA sequence encoding the polypeptide, the transcription of which is activated by the transcriptional activator.

In another embodiment, the host cell is a mutant of a parent fungal cell in which the mutant comprises (a) a modification or inactivation of a transcriptional activator of the present invention or a regulatory sequence thereof, and (b) an inducible promoter operably linked to a nucleic acid sequence encoding a transcriptional activator of the present invention and a promoter sequence to which the transcriptional activator can bind, operably linked to a nucleic acid sequence encoding the polypeptide, wherein (i) and (ii) can be introduced simultaneously or sequentially.

The host cells of the present invention are cultivated in a nutrient medium suitable for production of the polypeptide of interest using methods known in the art. For example, the cells may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art (see, *e.g.,* Bennett, J.W. and LaSure, L., eds., More Gene Manipulations in Fungi, Academic Press, CA, 1991). Suitable media are available from commercial suppliers or may be prepared using published compositions (*e*.*g*., in catalogues of the American Type Culture Collection). If the polypeptide is secreted into the nutrient medium, the polypeptide can be recovered directly from the medium. If the polypeptide is not secreted, it is recovered from cell lysates.

The resulting polypeptide may be isolated by methods known in the art. For example, the polypeptide may be isolated from the nutrient medium by conventional procedures including, but not limited to, centrifugation, filtration, extraction, spray drying, evaporation, or precipitation. The isolated polypeptide may then be further purified by a variety of procedures known in the art including, but not limited to, chromatography (*e*.*g*., ion exchange, affinity, hydrophobic, chromatofocusing, and size exclusion), electrophoretic procedures (*e*.*g*., preparative isoelectric focusing, differential solubility (*e*.*g*., ammonium sulfate precipitation), or extraction (see, *e*.*g*., Protein Purification, J.-C. Janson and Lars Ryden, editors, VCH Publishers, New York, 1989).

The polypeptide may be detected using methods known in the art that are specific for the polypeptide. These detection methods may include use of specific antibodies, formation of an enzyme product, disappearance of an enzyme substrate, or SDS-PAGE. For example, an enzyme assay may be used to determine the activity of the polypeptide. Procedures for determining enzyme activity are known in the art for many enzymes.

In one embodiment, the host cell produces at least about 20% more, preferably at least about 50% more, more preferably at least about 100% more, even more preferably at least about 200% more, and most preferably at least about 300% more of the polypeptide than a corresponding parent cell when cultivated under the same conditions.

The polypeptide may be any polypeptide whether native or heterologous to the mutant filamentous fungal cell. The term "heterologous polypeptide" is defined herein as a polypeptide, which is not produced by a cell. The term "polypeptide" is not meant herein to refer to a specific length of the encoded produce and therefore encompasses peptides, oligopeptides and proteins. The polypeptide may also be a recombinant polypeptide, which is a polypeptide native to a cell, which is encoded by a nucleic acid sequence, which comprises one or more control sequences, foreign to the nucleic acid sequence, which are involved in the production of the polypeptide. The polypeptide may be a wild-type polypeptide or a variant thereof. The polypeptide may also be a hybrid polypeptide, which contains a combination of partial or complete polypeptide sequences obtained from at least two different polypeptides where one or more of the polypeptides may be heterologous to the cell. Polypeptides further include naturally occurring allelic and engineered variations of the above-mentioned polypeptides.

In a preferred embodiment, the polypeptide is an antibody or portions thereof, an antigen, a clotting factor, an enzyme, a hormone or a hormone variant, a receptor or portions thereof, a regulatory protein, a structural protein, a reporter, or a transport protein.

In a more preferred embodiment, the enzyme is an oxidoreductase, transferase, hydrolase, lyase, isomerase, or ligase.

In an even more preferred embodiment, the enzyme is an aminopeptidase, amylase, carbohydrase, carboxypeptidase, catalase, cellulase, chitinase, cutinase, deoxyribonuclease, dextranase, esterase, alpha-galactosidase, beta-galactosidase, glucoamylase, alpha-glucosidase, beta-glucosidase, haloperoxidase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

In another even more preferred embodiment, the polypeptide is human insulin or an analog thereof, human growth hormone, erythropoietin, or insulinotropin.

The nucleic acid sequence encoding a heterologous polypeptide may be obtained from any prokaryotic, eukaryotic, or other source. For purposes of the present invention, the term "obtained from" as used herein in connection with a given source shall mean that the polypeptide is produced by the source or by a cell in which a gene from the source has been inserted.

The mutant filamentous fungal cells may also be used for the recombinant production of polypeptides, which are native to the cell. The native polypeptides may be recombinantly produced by, *e*.*g*., placing a gene encoding the polypeptide under the control of a different promoter to enhance expression of the polypeptide, to expedite export of a native polypeptide of interest outside the cell by use of a signal sequence, and to increase the copy number of a gene encoding the polypeptide normally produced by the cell. The present invention also encompasses, within the scope of the term "heterologous polypeptide", such recombinant production of polypeptides native to the cell, to the extent that such expression involves the use of genetic elements not native to the cell, or use of native elements which have been manipulated to function in a manner that do not normally occur in the filamentous fungal cell. The techniques used to isolate or clone a nucleic acid sequence encoding a heterologous polypeptide are known in the art and include isolation from genomic DNA, preparation from cDNA, or a combination thereof. The cloning of the nucleic acid sequences from such genomic DNA can be effected, *e*.*g*., by using the well known polymerase chain reaction (PCR), see, for example, Innis et al., 1990, PCR Protocols: A Guide to Methods and Application, Academic Press, New York. The cloning procedures may involve excision and isolation of a desired nucleic acid fragment comprising the nucleic acid sequence encoding the polypeptide, insertion of the fragment into a vector molecule, and incorporation of the recombinant vector into the mutant fungal cell where multiple copies or clones of the nucleic acid sequence will be replicated. The nucleic acid sequence may be of genomic, cDNA, RNA, semisynthetic, synthetic origin, or any combinations thereof.

In the methods of the present invention, heterologous polypeptides may also include fused or hybrid polypeptides in which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator. The hybrid polypeptides may comprise a combination of partial or complete polypeptide sequences obtained from at least two different polypeptides wherein one or more may be heterologous to the mutant fungal cell. An isolated nucleic acid sequence encoding a heterologous polypeptide of interest may be manipulated in a variety of ways to provide for expression of the polypeptide. Expression will be understood to include any step involved in the production of the polypeptide including, but not limited to, transcription, post-transcriptional modification, translation, post-translational modification, and secretion. Manipulation of the nucleic acid sequence encoding a polypeptide prior to its insertion into a vector may be desirable or necessary depending on the expression vector. The techniques for modifying nucleic acid sequences utilizing cloning methods are well known in the art.

The present invention is further described by the following examples, which should not be construed as limiting the scope of the invention.

### EXAMPLES

### Materials

Chemicals used as buffers and substrates were commercial products of at least reagent grade.

### Strains

AB4.1: a strain of *Aspergillus niger* which is a *cspA1 ρyrG1* derivative of strain ATTC 9029 (van Hartingsveldt, W., et al., 1987. Mol. Gen. Genet. 206:71-75; Bos, C.J., et al., Curr. Genet. 14:437-443)
AB1.13: a protease deficient strain of *Aspergillus niger* derived from UV mutagenesis of AB4.1 (Mattern, I.E., et al., 1992. Mol. Gen. Genet. 234:332-336)
13PAP2: an AB1.13 derivative containing multiple copies of the *A. nidulans amdS* gene (Corrick, R. A., et al, 1987. Gene 53: 63 - 71) under control of the *pepA* promoter of *A. niger* (Jarai G. and Buxton F. 1994. Curr Genet 26:238-244). The strain has a protease deficient phenotype and is unable to grow on medium containing acetamide as the sole nitrogen source. Strain 13PAP2 has been deposited at DSM under the name DSM No. 12298.
4PAP6: an AB4.1 derivative containing multiple copies of the of *A. nidulans amdS* gene under control of the *pepA* promoter of *A. niger*. The strain does not have a protease deficient phenotype and is able to grow on medium containing acetamide as the sole nitrogen source.
N402: a strain of *Aspergillus niger*, deposited at the ATCC (Manassas VA, USA) as ATCC Number: 64974
MC1046: a strain of *E*. *coli,* deposited at the ATCC as ATCC Number: 35467
*A.oryzae* IFO4177: available from Institute for Fermentation, Osaka; 17-25 Juso Hammachi 2-Chome Yodogawa-Ku, Osaka, Japan.
HowB101: described in WO 97/35956.

### Plasmids

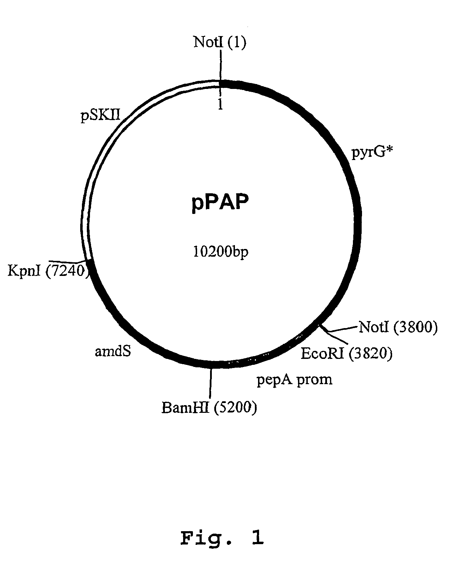

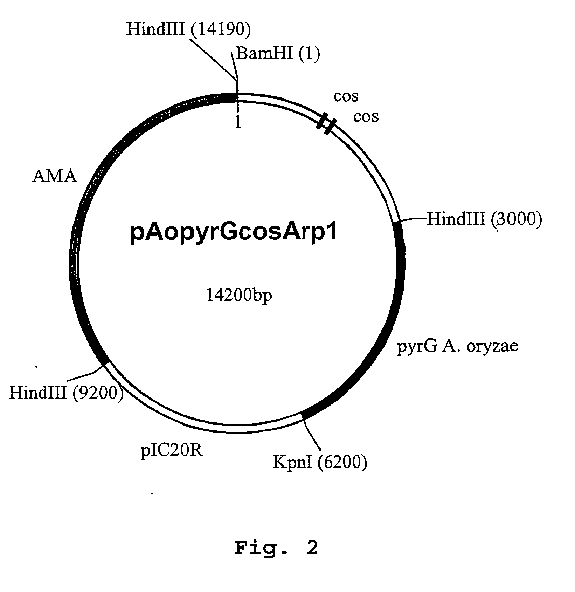

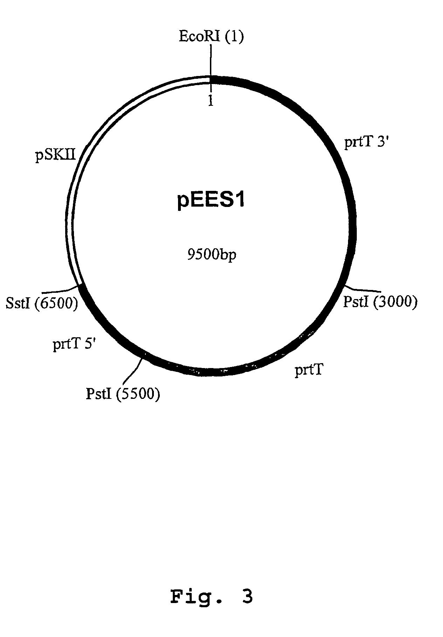

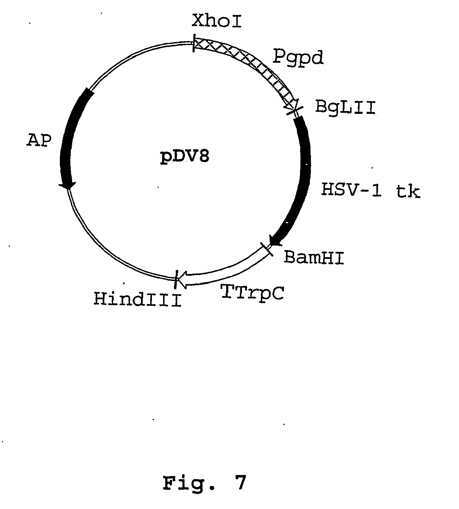

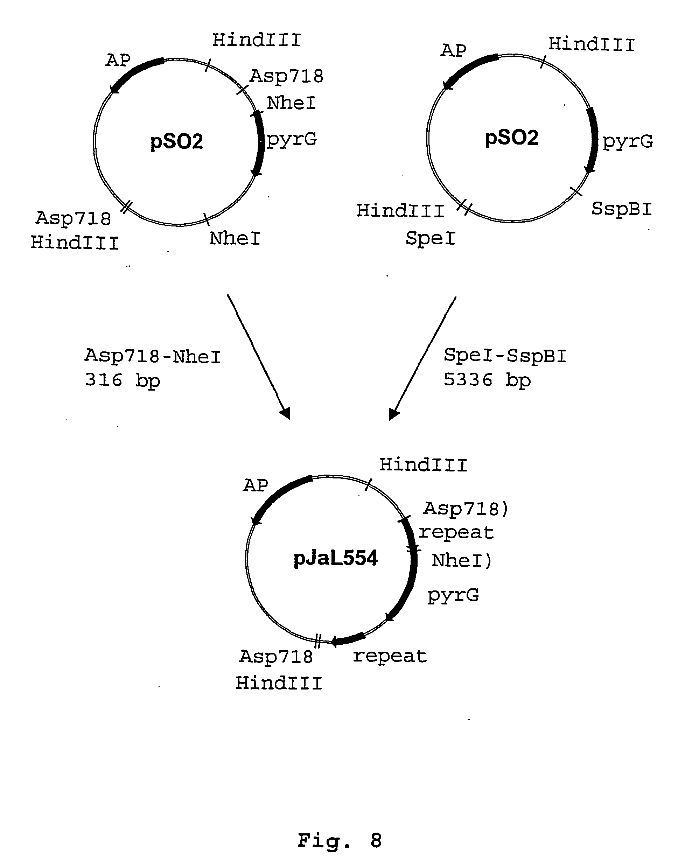

pPAP: constructed as described below in Example 1 and shown in Figure 1
pAopyrGcosArp1: constructed as described below in Example 1 and shown in Figure 2
pEES1: constructed as described below in Example 1 and shown in Figure 3
p3SR2: contains the *A. nidulans amdS* gene as described by C.M. Corrick, A.P. Twomey, and M.J. Hynes (1987. Gene 53: 63-71)
pABPYRG*-Not: contains an inactivated *pyrG* gene as described by Verdoes, J.C., et al. (1994. Gene 145: 179-187)
pHelp1: contains the *pyrG* gene from *A. oryzae* as a selective marker and the AMA1 sequences which enable autonomous replication in *A. niger*, cloned into the *E. coli* vector pIC20R, as described by Gems, D., et al. (1991. Gene 98: 61-67)
pAnscos1: contains two *cos* sites as described by Osiewacz, H.D. (1994. Curr. Genet. 26: 87-90)
pAO4-2: contains the *A. oryzae pyrG* gene as described by De Ruiter-Jacobs, Y.M.J.T., et al. (1989. Curr. Genet. 16: 159-163)
pAO4-13: contains the *A. oryzae pyrG* gene as described by De Ruiter-Jacobs, Y.M.J.T., et al. (1989. Curr. Genet. 16:159-163)
pUC19: as described by Yanisch-Perron, C., Vieira, J. and Messig, J. (1985, Gene 33:103-119)
pDV8: described in Example 8 and shown in Fig. 7.
pJaL554: described in Example 8 and shown in Fig. 8

### Deposit of Biological Materials

The following biological material has been deposited under the terms of the Budapest Treaty with the Deutsche Sammlung von Microorganismen und Zellkulturen GmbH, Mascheroder Weg 1b, D-38124 Braunschweig, Germany and given the following accession number:

| Deposit | Accession Number | Date of Deposit |
|---|---|---|
| *Escherichia coli*, pEES | DSM 12294 | 1998-07-14 |
| *Aspergillus niger* 13PAP2 | DSM 12298 | 1998-07-14 |

The strain has been deposited under conditions that assure that access to the culture will be available during the pendency of this patent application. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

### EXAMPLES

### Example 1

### Cloning of the A. niger prtT transcriptional activator

The *prtT* gene was cloned from 13PAP2, an *A. niger* mutant strain which is unable to express the *amdS* gene regulated by the *pepA* protease gene promoter and has a protease deficient phenotype (prt⁻).

### Construction of the A. niger 13PAP2 reporter strain

The plasmid pPA1 was contructed by ligation of the following three fragments:
1) the E. *coli* vector pBlueScript II SK (Stratagene Cloning Systems, La Jolla CA, USA) digested with EcoRI and KpnI;
2) a 1.4 kb EcoRI/BamHI restriction fragment containing the 1.2 kb promoter region of the *pepA* gene linked to about 130 bp of the *amdS* coding sequence from the start codon to an internal BamHI site, amplified by PCR; and
3) a 2.1 kb BamHI/KpnI fragment from p3SR2 which contains most of the *A. nidulans amdS* gene

Fragment 2 was constructed in two steps. In a first step genomic DNA from *A. niger* N402 prepared from protoplasts as described below in the section "Construction of the Cosmid Library" was used as the template, and the two oligonucleotides shown below, pepApr and pepA/amdS, were used as primers:
PepApr: CGG AAT TCG CAT GCT GGA GGT GCT TCT AA (SEQ ID NO: 6)
pepA/amdS:TTC CCA GGA TTG AGG CAT TTT GAC CAC GAG AAT (SEQ ID NO: 7)

The 1200 bp PCR product obtained from this reaction was then used as a primer in a second PCR reaction together with the oligonucleotide MBL1213 shown below, and plasmid p3SR2 as the template.
MBL1213: TAA CTT CCA CCG AGG TC (SEQ ID NO: 8)

The product obtained by ligation of the three fragments described above was subsequently transfected into *E. coli* DH5α.

In the final construction procedure, pPA1 was digested with NotI and ligated to a 3.8 kb NotI fragment from pABPYRG*-Not, resulting in plasmid pPAP which is shown in Fig. 1. pPAP was transformed into *A. niger* AB 1.13, and a transformant with pPAP integrated into the *pyrG* locus in multicopy was isolated. A spontanous 5 flourotic acid (FOA) resistant, uridine-requiring mutant of this transformant that could be complemented with the *pyrG* gene was named 13PAP2.

### Construction of pAopyrGcosArp1

The plasmid pAopyrGcosArp1 was constructed by ligation and subsequent transfection into *E. coli* DH5α of the following three fragments:
1) the *E. coli* vector pHelp1 cut with Acc65I and BamHI
2) a 3.0 kb BamHI/HindIII fragment from pAnscos1 containing two *cos* sites
3) a 3.2 kb Acc65I/HindIII fragment from pA04-2 containing the *A. oryzae pyrG* gene

The resulting plasmid, pAopyrGcosArp1, is self-replicating in *Aspergilli* and can be selected for by growth on medium lacking uridine. pAopyrGcosArp1 is depicted in Fig. 2.

### Construction of the cosmid library.

A cosmid library of *Aspergillus niger* was constructed using the "SuperCos1 cosmid vector kit" (Stratagene Cloning Systems, La Jolla CA, USA) according to the supplier's instructions.

Genomic DNA from *A. niger* N402 was prepared from protoplasts made by standard procedures.

After isolation the protoplasts were pelleted by centrifugation at 2000 rpm for 10 minutes in a Beckman GS-6R; the pellet was then suspended in a buffer containing 22.5 mM triisonaphtalene sulphonic acid, 275 mM para-aminosalicylic acid, 0.2 M Tris-HCl (pH 8.5), 0.25 M NaCl and 50 mM EDTA immediately followed by addition of 1 volume of phenol/chloroform (1:1). After careful mixing and centrifugation at 3000 rpm for 20 minutes the aqueous phase was decanted and DNA was precipitated using standard procedures.

The size of the genomic DNA was analysed by electrophoresis on a 0.3% agarose gel run for 20 hours at 30 volts at 4°C. The ethidium bromide stained gel showed that the recovered DNA ranged in size from 50 to greater than 100 kb. The DNA was partially digested using MboI. The size of the digested DNA was 30 to 50 kb as determined by the same type of gel analysis as above. The pAopyrGcosArp1 vector, purified using a kit from QIAGEN (Venlo, The Netherlands) following the manufacturer's instructions, was digested with BamHI, dephosphorylated and gel purified. Ligation and packaging were performed following standard procedures.

After titration of the library, all of the packaging mix from a single ligation and packaging was transfected into the host cell, MC1046, and plated on 50 µg/ml ampicillin LB plates. Approximately 40,000 colonies were obtained. Cosmid preparations from 10 colonies showed that they all had inserts of the expected size. The 40,000 colonies were then soaked in LB medium and scraped off of the plates, then aliquoted for storage in 15% glycerol at -80°C. This represents an approximate 40-fold amplification of the *A. niger* genome.

### Selection of A. niger prtT clones

Cosmid DNA was prepared from the library and introduced into 13PAP2 according to the transformation procedure described by P.J. Punt and C.A.M.J.J. Van Den Hondel (1992. Methods Enzymol 216: 447-457). Repeated efforts to select for the *pyrG* marker only resulted in a recovery of between 4000 to 30,000 transformants. A double selection for the *pyrG* marker and growth on medium containing acetamide as the sole nitrogen source resulted in a total of 65 primary transformants from five different experiments.

Each primary transformant was screened for protease activity, growth on medium containing acetamide as the sole nitrogen source and instability of these two characteristics. An acetamidase⁺ phenotype, screened by growth on medium containing acetamide, is an indication of acetamidase activity resulting from activation of the *pepA* promoter in the reporter cassette in which the *pepA* promoter is linked to the *amdS* coding sequence. A protease⁺ phenotype was screened using minimal medium plates containing dialyzed skim milk as the sole nitrogen source (Mattern, I.E., et al., 1992. Mol Gen Genet 234:332-336). On these plates the wild-type AB4.1 strain makes a clear halo whereas the AB1.13 mutant produces a very small halo. This difference is not due to differences in the activity of *pepA* since a *pepA* deleted strain can also produce a large halo on these plates. Therefore, a large halo on milk plates indicates activation of other extracellular proteases.

Instability was tested by growing diluted spore stocks on a medium containing uridine. Single-spore-derived colonies were picked from these plates and tested for protease activity and growth on acetamide. The screening results revealed that in more than 70% of the colonies both characteristics were lost. Therefore, the two phenotypes were either lost or retained together, indicating that activation of the *pepA* promoter and other protease promoters is coordinately regulated and linked to the presence of the *pyrG* marker. The gene responsible for this phenotype was named *prtT.* Twelve acetamidase⁺, protease⁺ transformants were then isolated.

### Isolation of the A.niger prtT gene

In order to rescue the *prtT* gene from the acetamidase⁺, protease⁺ transformants of 13PAP2, DNA was prepared from mycelium grown in minimal medium as previously described. This DNA was used in an attempt to transform competent *E. coli* DH5α cells. Several hundreds of ampicillin-resistant colonies were obtained. DNA analysis showed they all contained sequences derived from the pHelp1 plasmid. Cosmid DNA isolated from *E. coli* colonies was then retransformed into 13PAP2. Two DNA samples gave rise to transformants, which showed both growths on acetamide containing medium and increased protease activity. DNA from one of the cosmids, ACR1, was then digested with several restriction enzymes. The resulting fragments were then co-transformed with pAopyrGcosArp1 into strain 13PAP2. EcoRI, PstI, BamHI and KpnI digestion of ACR1 gave rise to transformants capable of growth on acetamide and high protease activity, whereas SalI and HindIII digests did not. Because EcoRI digestion gave the simplest pattern, separate EcoRI fragments were gel-isolated and with pAopyrGcosArp1 used to co-transform 13PAP2. Only one fragment, a 15 kb EcoRI fragment, gave rise to transformants capable of growth on acetamide-containing medium. This fragment was subcloned in pBluescript II SK in order to subclone *prtT* from the cosmid. Since the insert of this clone was still rather large, separate PstI bands were gel isolated and each was co-transformed with pAopyrGcosArp1 into 13PAP2. Only one band, a 2.5 kb PstI fragment, gave rise to transformants that could grow on acetamide-containing medium. This fragment was subcloned in pBlueScript II SK. Four subclones, ClE 0.7, ClE 1.8, NcE 1.1 and NcE 1.4, were constructed from this plasmid based on the restriction map. In addition, a 6.5. kb SstI/EcoRI fragment encompassing the 2.5 kb PstI fragment was subcloned, resulting in pEES1 (shown in Fig. 3).

Southern blot analysis of genomic DNA from AB4.1 showed the presence of only one copy of *prtT*.

### Example 2

### Sequencing of the A. niger prtT gene and analysis of the sequence

All sequence reactions were prepared using dRhodamine Terminator Cycle Sequencing Kits or BigDye™ Terminator Cycle Sequencing Kits from the Perkin-Elmer Corporation (Branchburg NJ, USA). The reactions were run on an ABI PRISM^{®} 377 DNA Sequencer (Perkin-Elmer Corporation) following the manufacturer's instructions.

The *prtT* gene was sequenced from the genomic clones ClE 0.7, ClE 1.8, NcE 1.1, NcE 1.4 and pEES1. The sequence specific primers used are listed below:
122958: CGA TCG ATG ACT GCC TGT (SEQ ID NO: 9)
122956: AGA GAC ACA TAG TGC CTT (SEQ ID NO: 10)
122959: GCT TAT AGT CGA TAG CGC (SEQ ID NO: 11)
122960: CCT CTC TCC AGC GAT GGT (SEQ ID NO: 12)
122962: ATG GAA TAC ATA CTG CTT (SEQ ID NO: 13)
122961: ATG AAA CCC ACT GTA GCT (SEQ ID NO: 14)
122963: TGC TCG ATA AGC GGG TCC (SEQ ID NO: 15)
122964: AAT CTT ATG GAC CCG CTT (SEQ ID NO: 16)
124289: CCC CGG GAA ACA AGA ACA GG (SEQ ID NO: 17)
124290: GTT GGC GGA CCT TGA CTA TG (SEQ ID NO: 18)
125112: ACA GCT ACA GTG GGT TTC ATC T (SEQ ID NO: 19)
125111: AGT CAA CGG GGG AAG TCT C (SEQ ID NO: 20)
128330: CTA GCA GCG TAT CGG TCA GC (SEQ ID NO: 21)
130887: CTT GGA AAA GAA ACG ATA G (SEQ ID NO: 22)
130888: AAC GTA CGC TTT CCT CCT T (SEQ ID NO: 23)
134135: GGG TCC GTC CAG TCC GTT CTT (SEQ ID NO: 24)

- 48 reverse: AGC GGA TAA CAA TTT CAC ACA GGA (SEQ ID NO: 25)
- 40 universal: GTT TTC CCA GTC ACG AC (SEQ ID NO: 26)

A mutant allele of the gene was obtained by PCR amplification of genomic DNA isolated from the mutant strain AB1.13 using the following primers:
PstI: TC ATC CCT GGT GTT ACT GC (SEQ ID NO: 27)
PstII: C ATG GAT TGG CTG GCC G (SEQ ID NO: 28)

The complete DNA sequence of the *prtT* gene is shown in SEQ ID NO:1. The sequence of the PCR fragment of the mutant allele is shown in SEQ ID NO:4.

Analysing the DNA sequence SEQ ID NO:1 using the computer software Netgene 2 (S.M. Hebsgaard, P.G. Korning, N. Tolstrup, J. Engelbrecht, P. Rouze, S. Brunak (1996. Nucleic Acids Research 24: 3439-3452) suggested the existence of 5 exons (see annotations to SEQ ID NO 1).

### Analysis of the A. niger prtT cDNA

mRNA was purified from total RNA (isolated according to the DNA isolation method described above in Example 1) using a commercial poly(A)⁺ RNA isolation kit (Pharmacia, Uppsala SE) from a culture of *A. niger* grown under conditions favourable for protease production (J.P.T.W. Van Den Hombergh, et al., 1997. Eur. J. Biochem. 247:605-613). Double stranded cDNA was prepared using standard procedures and used for PCR reactions with the following primers:
oligo-dT primer: T₂₀N
   Prt270n: TACTCTCCAGATTGCCTG (SEQ ID NO: 29)
   Prt1420r: TGAGATACCACTCAGCAG (SEQ ID NO: 30)
   prt1350n: TGCACTTCTCTGTCTCTG (SEQ ID NO: 31)
   Prt2365r: GACTTCTGGCATCAGTTG (SEQ ID NO: 32)
   prt2320n: CTCATGGATGGCATGATC (SEQ ID NO: 33)

A PCR reaction with the primers Prt270n and Prt1420r produced a fragment of approximately 1.0 kb. The fragment was cloned into a pGEM-T vector (Promega Corp., Madison WI, USA), and the insert in the resulting plasmid was sequenced using the primers 122958, 122960, -40 universal and -48 reverse. The result confirmed the presence of two introns in this part of the gene.

A second PCR reaction with the primers Prt1350n and Prt2365r produced a fragment of approximately 0.9 kb. This fragment was also cloned in a pGEM-T vector, and the insert in the resulting plasmid was sequenced using the primers 124289, 124290, -40 universal and -48 reverse. The result confirmed the presence of a single intron in this part of the gene.

Another PCR reaction with the oligo-dT primer and primer Prt2320n produced a fragment of approximately 350 bp. This fragment was also cloned in a pGEM-T vector. Sequencing of the insert using primers -40 universal and -48 reverse showed that the fragment contained the 3' part of *prtT* and confirmed the presence of another intron.

The deduced protein sequence of the translated *prtT* gene is shown in SEQ ID NO:2. The deduced protein sequence of the translated mutant allele *prt13* is shown in SEQ ID NO:5. A comparison of SEQ ID NO:2 and SEQ ID NO:5 indicates that the only difference between the two is in position 112 where the leucine residue in the translated *prtT* gene is replaced by proline in the translated *prt13* gene.

Analysis of the deduced PrtT protein sequence reveals the presence of a Zinc(II)2Cys6 binuclear cluster DNA binding motif (SEQ ID NO:2, residues 47-81). This motif defines the GAL4 class of fungal transcriptional activators (Reece, M. J., and Ptashne, M. 1993. Science 261: 909-911). The presence of the motif in the *prtT* gene strongly indicates that prtT is a transcriptional activator.

### EXAMPLE 3:

### Disruption of the prtT gene in a wild-type A. niger strain

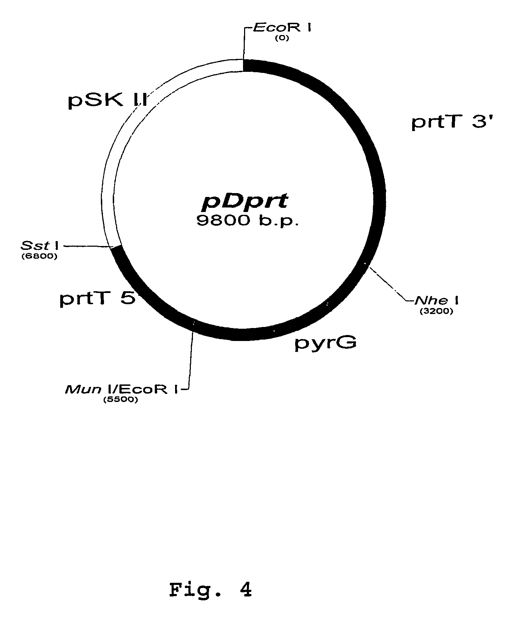

A plasmid was constructed in which the upstream and downstream sequences of the *prtT* gene are separated by the *A. oryzae pyrG* gene. Plasmid pEES1 was digested with MunI and NheI, which removed a 2.1 kb fragment containing most of the coding sequence of *prtT*. A 2.3 kb EcoRI/NheI fragment from pA04-13 containing the *A. oryzae pyrG* gene was cloned in the MunI and NheI sites of pEES1. The resulting plasmid, shown in Fig. 4, was named pDprt. This construct was then used to transform *A. niger* strain AB4.1 to uridine prototrophy. About 150 uridine prototrophic transformants were then analyzed for protease activity on skim milk containing plates. Five of these did not make a halo on these plates indicating that protease activity was very low. Comparison of strains with a disrupted *prtT* gene and the mutant AB1.13 strain did not show any differences in protease activity or phenotype.

### Example 4

### Overexpression of A. niger PrtT

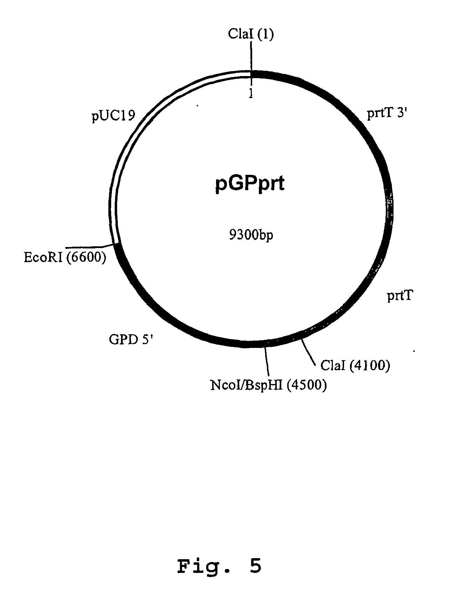

A plasmid, pGPprt, (Figure 5) containing the coding region and 3' noncoding sequences of *prtT* fused to the promoter of the *A. niger gpd* gene was constructed. The *gpd* gene codes for glyceraldehyde-3-phosphate dehydrogenase and a constitutively expressed enzyme involved in primary metabolism. The promoter used was a fragment upstream of the coding region.
The plasmid is transformed into *A. niger* AB4.1 by cotransformation with the pyrG selection plasmid pA04-13. Transformants with increased prtT transcription as determined by Southern blot analysis is analysed for increased protease expression.

### Example 5

### Isolation of the Zn²⁺-finger from the A. oryzae prtT gene

The *A. niger prtT* gene is shown in SEQ ID NO: 1. The protein sequence deduced from the DNA sequence of *prtT* (SEQ ID NO: 2) contains a so called Zn²⁺-finger motif expected to be responsible for the DNA binding of the transcriptional activator encoded by *prtT*. The Zn²⁺-finger motif has the following amino acid sequence: Met Thr Ala Cys His Thr Cys Arg Lys Leu Lys Thr Arg Cys Asp Leu Asp Pro Arg Gly His Ala Cys Arg Arg Cys Leu Ser Leu Arg Ile Asp Cys (SEQ ID NO: 34).

Degenerate primers able to code for amino acid sequences from the motif were designed and synthesized by DNA Technology A/S, Forskerparken, Gustav Wieds vej 10, DK-8000 Aarhus C, Denmark. The primers had the following sequences:

The primers were used in a PCR reaction with genomic *A. oryzae* IFO4177 DNA as template. The reaction was performed in a total volume of 100 µl containing 154 pmol of primer 137396 and 10164 pmol of primer 137397. 30 PCR cycles with 56°C as annealing temperature and 30 seconds elongation time were run. Another PCR reaction using *A. niger* genomic DNA and the primers 137394: ATGACTGCCTGTCACACATG (SEQ ID NO: 37)
and 137395: AGACAGCGACGGCACGCATG (SEQ ID NO:38), which are specific for the *A. niger prtT* gene, was also run. In this reaction 10 pmol of each primers was used in a 100 µl reaction. Aliquots of the two reactions were applied to a 3% agarose gel. After electrophoresis three approximately equally intense bands could be seen in the *A. oryzae* reaction and two bands in the A. *niger* reaction. One of the bands in the *A. niger* reaction was more intense than the other and further had the expected size. One of the *A. oryzae* bands had the same size as the most intense *A. niger* band and was isolated from the gel. The fragment was cloned into the vector pCR2.1 (Invitrogen™). Plasmids from two individual colonies were sequenced. The sequences are shown in figure 1. The two sequences differ at the end reflecting their origin in different degenerate primers. They are identical in the middle 40bp, which are amplified from the genomic DNA. These 40 basepairs encode a polypeptide identical to a part of the Zn²⁺-finger of the *A. niger prtT* gene.

### Example 6

### Isolation of the N-terminal of the A. oryzae prtT gene

The inverse PCR method was used to isolate the *A. oryzae prtT* gene. The primers 144428: CACCGAGTTTTAAGCTTGCGG (SEQ ID NO: 39) and 144429:GCGATCTTGATCCACGAGGG (SEQ ID NO: 40) were synthezised by DNA Technology A/S (Denmark). Genomic DNA was cut with a number of restriction enzymes and religated. The ligation mixtures were used as templates in PCR reactions with the primers 144428/144429. In a reaction with *BamHI* restricted and religated DNA as template a fragment of approximately 2.5kb was observed after electrophoresis on an agarose gel. The fragment was labelled with ³²P by the random priming method and used as a probe against a filter containing a gridded cosmid library of genomic *A. oryzae* DNA. The construction of the library is described in WO 98/01470. The cosmid 11F8 showed a positive hybridization signal with the probe. A Southern blot containing DNA from 11F8 and genomic DNA restricted with *BamHI, EcoRI, PstI* or *XhoI* was probed with the 2.5kb inverse PCR fragment. The size of hybridizing bands from genomic DNA were compared with those from the cosmid DNA. Apparently some rearrangement of the cosmid had occurred since only a minority of the bands from the genomic DNA had counterparts in the cosmid. Two hybridizing fragments from the cosmid, a 1.2 kb *EcoRI* fragment and a 1.0 kb *PstI* fragment, looked equal in size to hybridizing genomic fragments. The two fragments were sub-cloned from the cosmid and sequenced. Analysis of the sequence data showed that the fragments overlap. In total 1497 bp of sequence was obtained. Oligonucleotides encoding the Zn²⁺-finger were not contained within the sequence. A *BamHI* site was found close to one end of the sequence in a region only covered by the *EcoRI* sub-clone, thus allowing the position of the sequenced genomic fragment relative to the Zn²⁺-finger to be determined. The primer 153468: CGGGATGAATTGTAGAGAGGC (SEQ ID NO: 41) was prepared by DNA Technology A/S (Denmark). The primer sequence is contained within the 1497 bp fragment. It is found at the end closest to the Zn²⁺-finger and points in that direction. Two primers both of *prtT* Zn²⁺-finger specific sequence and pointing either downstream (140358) or upstream (140359) were also prepared by DNA Technology A/S (Denmark). The sequence of the two primers are as follows: 140358: CGCAAGCTTAAAACTCGGTGGGATC (SEQ ID NO: 42) and 140359: CCTCGTGGATCAAGATCGCA (SEQ ID NO: 43). Two PCR reactions, one with the primers 153468 and 140358 and one with 153468 and 140359, respectively, were performed with genomic DNA as template. The reaction with 153468 and 140359 gave a band of approximately 1.1 kb, the other reaction gave no visible bands, when analysed on an agarose gel. The 1.1 kb fragment was cloned into pCR4Blunt-TOPO (Invitrogen) and sequenced. The fragment contained part of the Zn²⁺-finger and overlaps with the 1497bp fragment. Translation of the sequence showed that the region immediately upstream of the Zn²⁺-finger encodes a polypeptide with homology to the N-terminal of *prtT* from *A. niger*.

### Example 7

### Isolation of the complete A. oryzae prtT gene

The remaining parts of the gene were cloned by two consecutive rounds of inverse PCR. In the first inverse PCR reaction the genomic DNA was restricted with *EcoRV* and re-ligated. The PCR reaction was run with the primers 175653: GATGAAAAGAATAATCGGCGAG (SEQ ID NO: 44) and 175654: CGCGGCACACTACCCCCGTTG (SEQ ID NO: 45). The reaction resulted in the synthesis of a 1.9kb fragment, which was cloned into the pCR4Blunt-TOPO vector and sequenced. Analysis of the sequence data showed that the fragment contains a gene with homology to the *A. niger prtT* gene and that the 3'end of the gene was missing. The second inverse PCR reaction was thus performed. The primers were B0403G08: ATCTAGCTCAAGCATTAGCGGC (SEQ ID NO: 46) and B0403G09: AATTTCGGCCCTTTAGTGTCC (SEQ ID NO: 47). *BglII* restricted and re-ligated genomic DNA was used as template. A 2.4 kb fragment was obtained and cloned into the pCR4Blunt-TOPO vector and sequenced. Analysis of the sequence showed that the complete *A. oyzae prtT* gene had been obtained. The DNA sequence of the *A. oryzae prtT* gene is shown in SEQ ID NO: 48 and the deduced amino acid sequence of the encoded protein is shown in SEQ ID NO: 49.

### Example 8

### Disruption of the Aspergillus oryzae prtT gene.

The *A. oryzae prtT* gene was disrupted using a method of positive/negative selection. A disruption cassette consisting of 2kb of the *A. oryzae prtT* gene (SEQ ID NO: 48) with an insertion of the *pyrG* gene in the middle is cloned into a vector (pDV8) containing the herpes simplex virus I tymidine kinase gene (HSV-tk) flanked by fungal expression signals. Expression of the tymidine kinase gene makes the host sensitive to 5-flouro-2-deoxyuridine. A disrupted strain can be isolated by positive selection for the *pyrG* gene in a *pyrG* minus host and deselection of the tymidine kinase gene on 5-flouro-2-deoxyuridine. Since the tymidinie kinase gene and the *pyrG* gene are present in the same DNA fragment selection is for transformants in which a doubble cross-over event has happened. The system gives fewer transformants pr. Micro g of DNA than transformation with just a disruption cassette, but the frequency of transformants in which the desired homologous recombination event has occurred is much higher.
The *pyrG* gene used here is flanked by repeats enabling a later removal by selection for 5-fluoroorotic acid resistance. The *pyrG* gene is isolated from the plasmid pJaL554.

### The pDV8 plasmid:

pDV8 was kindly provided by Matthew S. Sachs, University of Oregon, PO Box 91000, Portland, OR 97291-1000, USA. pDV8 (Fig. 7) is a pSP65 (Promega™) based plasmid containing the HSV-tk gene on a 1.2 kb *BglII*/*BamHI* fragment inserted between a 1.0 kb *XhoI*/*BglII* fragment of the *A. nidulans gpd* promoter and a 0.8 kb *BamHI*/*HindIII* fragment containing the *A. nidulans trpC* transcriptional terminator.

The *A. nidulans gpd* promoter and the *trpC* transcriptional terminator are taken from the plasmid pAN51-2 (Punt et al., (1990), Gene 93, p.101-109). The HSV-tk gene is described by McKnight S.L., (1980), Nucleic Acids Res. 8:5949-5964, Database accession no. EMBL v00470, position 252-1479. The construction of pDV8 is described in Vaught-Alexander D (thesis) Expression of the herpes simplex virus type-1 thymidine kinase gene in *Neurospora crassa*, (1994), Oregon Graduate Institute of Science & Technology, University of Portland, PO Box 91000, Portland, OR 97291-1000, USA. The sequence of pDV8 is included in this application as SEQ ID NO: 50. Single-, double- and multicopy *A. oryzae* transformants of pDV8 were isolated by transforming a pDV8 derivative containing the *A. oryzae niaD* gene into an *A. oryzae niaD* mutant. The copy number of the HSV-tk gene was determined by Southern analysis. The transformants and the untransformed host were inoculated onto plates containing varying concentrations of 5-flouro-2'-deoxyuridine. From inspection of the growth on the plates it was decided to use 6 microM of 5-flouro-2'-deoxyuridine in the plates for future positive/negative selection experiments. At this concentration none of the pDV8 transformants grew, while the untransformed host was only slightly inhibited.

### Description of pJaL554:

PJaL554 was constructed by ligating the 316 bp *Asp718-NheI* fragment to the 5336 bp *SpeI-SspBI* fragment from the *pyrG* containing plasmid pSO2 (described in WO 97/35956). Thus, pJaL554 harbours the *A. oryzae pyrG* gene flanked by 316 bp repeats. The construction is illustrated in Fig. 8.

### Construction of a prtT disruption plasmid in the pDV8 vector:

A PCR reaction is performed on chromosomal *A. oryzae* IFO4177 DNA with the primers B1042E05 and B1450E07.
B1042E05: CGCGCGTATCCTATTGCC (SEQ ID NO: 51)
B1450E07: GCCGGAAATGTTGTACCTAC (SEQ ID NO: 52).
A fragment of 2078 basepairs is obtained and cloned into the pCR4Blunt-TOPO (Invitrogen™) vector. The resulting plasmid is sequenced with the standard M13 forward (-40) and reverse primers to ensure that the correct fragment is obtained. The PCR fragment is excised from the vector by the restriction enzyme *EcoRV* which cuts twice internally in the fragment. The cut sites are located at positions 1 and 1964 in SEQ ID NO: 48. The 1964 bp fragment is ligated with the pDV8 vector, which has been cut with *HindIII* and blunt ended by filling in the ends with the Klenow fragment of DNA polymerase I from *E. coli* and dNTP. The resulting plasmid is cut with *HindIII*, which is located in the *prtT* fragment (in the part encoding the Zn²⁺-finger) at position 962 in SEQ ID NO: 48, dephosphorylated and ligated with the *pyrG* gene isolated from pJaL554 as a 2.5 kb *HindIII* fragment.

### Selection of prtT disrupted strains:

The disruption plasmid described above was linearized with *NotI* and transformed into *A. oryzae* HowB101 (described in WO 97/35956), a *pyrG* minus derivative of IFO4177. The transformation is done essential as described in EP 0 238 023. Transformants are selected on plates containing Coves salt solution (Cove DJ, (1966), Biochim. Biophys. Acta 113:51-56), 1 M sucrose for osmotic stabilization and as carbon source, 20g/L agar, 10 mM NaNO₃ and 6 microM 5-flouro-2-deoxyuridine (Sigma). The transformants are reisolated once on the same type of plates. Transformants carrying a disrupted *prtT* gene are identified by Southern blot analysis.

A strain carrying the *prtT* disruption is used as host for expression of a truncated PDI gene (Protein Disulfide isomerase gene) harbored on the expression plasmid pCaHj445 (described in US patent 5,879,664). pCaHj445 is transformed into the *A. oryzae prtT* disrupted strain by cotransformation with the plasmid p3SR2 containing the *A. nidulans amdS* gene. Transformation and selection on acetamide plates is done essentially as described in EP 0 238 023. After reisolation the transformants are fermented in shake flasks or fermentors and the PDI protein is purified from the fermentation broth.

## Claims

1. A nucleic acid sequence, which comprises the DNA sequence shown in SEQ ID NO: 48, and encoding the polypeptide shown in SEQ ID NO: 49, which polypeptide has transcriptional activation activity.

2. A nucleic acid construct comprising the nucleic acid sequence of claim 1 operably linked to one or more control sequences, which direct the production of the polypeptide in a suitable expression host.

3. An expression vector comprising the nucleic acid construct of claim 2, a promoter, and transcriptional and translational stop signals.

4. A non-human host cell comprising the nucleic acid construct of claim 2 or the expression vector of claim 3.

5. A polypeptide which is encoded by the DNA sequence shown in SEQ ID NO: 48.

6. A method for producing the polypeptide of claim 5 comprising (a) cultivating the host cell of claim 4 under conditions conducive for production of the polypeptide; and (b) recovering the polypeptide.

7. A fungal host cell useful for the production of a polypeptide, wherein the cell:
a) is a mutant of a parent fungal cell in which the parent cell comprises one or more DNA sequences encoding a protease, the transcription of which is activated by a transcriptional activator encoded by a nucleic acid sequence of claim 1; and
b) produces less of the transcriptional activator and the protease(s) than the parent cell when cultured under the same conditions, and wherein the reduced production of the transcriptional activator is obtained by modification or inactivation of a nucleic acid sequence encoding the transcriptional activator shown in SEQ ID NO: 49, or by modification or inactivation of a control sequence required for the expression of the activator polypeptide.

8. The host cell of claim 7, wherein the control sequence is a promoter sequence, or a functional part thereof.

9. A fungal host cell useful for the production of a polypeptide, wherein the host cell is a mutant of a parent cell, in which the mutant:
a) produces more of a transcriptional activator encoded by a nucleic acid sequence of claim 1 than the parent cell when cultured under the same conditions, by introducing into the parent cell one or more copies of: (i) a nucleic acid sequence of claim 1, (ii) the nucleic acid construct of claim 2, or (iii) the expression vector of claim 3, and
b) comprises a DNA sequence encoding the polypeptide, the transcription of which is activated by the transcriptional activator.

10. The host cell of claim 9, wherein the nucleic acid sequence encoding the transcriptional activator is operably linked to a promoter, which is stronger than the corresponding promoter of the parent cell.

11. The host cell of claim 10, wherein the promoter mediates the expression of a gene encoding an extracellular protease.

12. A fungal host cell useful for the production of a polypeptide, wherein the cell is a mutant of a parent cell in which the mutant comprises:
a) a modification or inactivation of a transcriptional activator which is encoded in a native nucleic acid sequence of claim 1, or by modification or inactivation of a control sequence required for the expression of the activator polypeptide , and
b) (i) an inducible promoter operably linked to a nucleic acid sequence of claim 1, and (ii) a promoter sequence, which can be activated by the transcriptional activator encoded by the nucleic acid sequence of claim 1, operably linked to a nucleic acid sequence encoding the polypeptide, wherein (i) and (ii) can be introduced simultaneously or sequentially.

13. The host cell of claim 12, wherein the promoter sequence, or a functional part thereof, is from a protease gene.

14. A method of producing a polypeptide, comprising:
(a) cultivating the host cell of any of claims 7 to 13, wherein the host cell harbours a gene encoding the polypeptide, in a nutrient medium suitable for production of the polypeptide; and
(b) recovering the polypeptide from the nutrient medium of the mutant cell.

15. The method of claim 14, wherein the polypeptide is native to the parent cell or wherein the polypeptide is heterologous to the parent cell.

16. The method of claim 14-15, wherein the polypeptide is an antibody or portions thereof, an antigen, a clotting factor, an enzyme, a hormone or a hormone variant; a receptor or portions thereof; a regulatory protein; a structural protein; a reporter; or a transport protein.

## Patentansprüche

1. Nukleinsäuresequenz, welche die in SEQ ID NO: 48 gezeigte DNA-Sequenz umfasst, und kodierend das in SEQ ID NO: 49 gezeigte Polypeptid, welches Polypeptid transkriptionale Aktivationsaktivität aufweist.

2. Nukleinsäurekonstrukt, umfassend die Nukleinsäure gemäß Anspruch 1, funktionsfähig verknüpft mit einer oder mehreren Kontrollsequenzen, welche die Herstellung des Polypeptids in einem geeigneten Expressionswirt steuern.

3. Expressionsvektor, umfassend das Nukleinsäurekonstrukt gemäß Anspruch 2, einen Promotor und transkriptionale und translationale Stoppsignale.

4. Nichthumane Wirtszelle, umfassend das Nukleinsäurekonstrukt gemäß Anspruch 2 oder den Expressionsvektor gemäß Anspruch 3.

5. Polypeptid, welches durch die in SEQ ID NO: 48 gezeigte DNA-Sequenz kodiert ist.

6. Verfahren zum Herstellen des Polypeptids gemäß Anspruch 5, umfassend (a) Kultivieren der Wirtszelle gemäß Anspruch 4 unter Bedingungen, die für die Herstellung des Polypeptids förderlich sind; und (b) Gewinnen des Polypeptids.

7. Pilzwirtszelle, die für die Herstellung eines Polypeptids verwendbar ist, wobei die Zelle:
a) eine Mutante von einer Ausgangspilzzelle ist, in welcher die Ausgangszelle eine oder mehrere DNA-Sequenzen umfasst, die eine Protease kodieren, deren Transkription durch einen Transkriptionsaktivator, der durch eine Nukleinsäuresequenz gemäß Anspruch 1 kodiert ist, aktiviert ist; und
b) weniger von dem Transkriptionsaktivator und der/den Protease(n) herstellt als die Ausgangszelle, wenn unter den gleichen Bedingungen kultiviert, und wobei die reduzierte Herstellung des Transkriptionsaktivators durch Modifikation oder Inaktivierung einer Nukleinsäuresequenz, kodierend den in SEQ ID NO: 49 gezeigten Transkriptionsaktivator, oder durch Modifikation oder Inaktivierung einer Kontrollsequenz, die für die Expression des Aktivatorpolypeptids erforderlich ist, erhalten ist.

8. Wirtszelle nach Anspruch 7, wobei die Kontrollsequenz eine Promotorsequenz oder ein funktioneller Teil davon ist.

9. Pilzwirtszelle, verwendbar für die Herstellung eines Polypeptids, wobei die Wirtszelle eine Mutante einer Ausgangszelle ist, in welcher die Mutante:
a) mehr von einem Transkriptionsaktivator, der durch eine Nukleinsäuresequenz gemäß Anspruch 1 kodiert ist, als die Ausgangszelle herstellt, wenn unter den gleichen Bedingungen kultiviert, durch Einfügen von einer oder mehreren Kopien von: (i) einer Nukleinsäuresequenz gemäß Anspruch 1, (ii) dem Nukleinsäurekonstrukt gemäß Anspruch 2 oder (iii) dem Expressionsvektor gemäß Anspruch 3, in die Ausgangszelle, und
b) eine DNA-Sequenz umfasst, kodierend das Polypeptid, dessen Transkription durch den Transkriptionsaktivator aktiviert ist.

10. Wirtszelle nach Anspruch 9, wobei die den Transkriptionsaktivator kodierende Nukleinsäuresequenz funktionsfähig mit einem Promotor verknüpft ist, der stärker ist als der entsprechende Promotor der Ausgangszelle.

11. Wirtszelle nach Anspruch 10, wobei der Promotor die Expression eines Gens vermittelt, das eine extrazelluläre Protease kodiert.

12. Pilzwirtszelle, verwendbar für die Herstellung eines Polypeptids, wobei die Zelle eine Mutante einer Ausgangszelle ist, wobei die Mutante umfasst:
a) eine Modifikation oder Inaktivierung eines Transkriptionsaktivators, welcher in einer nativen Nukleinsäuresequenz gemäß Anspruch 1 kodiert ist, oder durch Modifikation oder Inaktivierung einer Kontrollsequenz, die für die Expression des Aktivatorpolypeptids erforderlich ist, und
b) (i) ein mit einer Nukleinsäuresequenz gemäß Anspruch 1 funktionsfähig verknüpfter induzierbarer Promotor und (ii) eine Promotorsequenz, welche durch den durch die Nukleinsäuresequenz gemäß Anspruch 1 kodierten Transkriptionsaktivator aktiviert werden kann, funktionsfähig verknüpft mit einer Nukleinsäuresequenz, die das Polypeptid kodiert, wobei (i) und (ii) simultan oder sequentiell eingeführt werden können.

13. Wirtszelle nach Anspruch 12, wobei die Promotorsequenz, oder ein funktioneller Teil davon, von einem Proteasegen ist.

14. Verfahren zum Herstellen eines Polypeptids, umfassend:
(a) Kultivieren der Wirtszelle nach einem beliebigen der Ansprüche 7 bis 13, wobei die Wirtszelle ein Gen inneträgt, das das Polypeptid kodiert, in einem zur Herstellung des Polypeptids geeigneten Nährmedium; und
(b) Gewinnen des Polypeptids aus dem Nährmedium der mutierten Zelle.

15. Verfahren nach Anspruch 14, wobei das Polypeptid gegenüber der Ausgangszelle nativ ist oder wobei das Polypeptid gegenüber der Ausgangszelle heterolog ist.

16. Verfahren nach Anspruch 14-15, wobei das Polypeptid ein Antikörper oder Teile davon ist, ein Antigen, ein Gerinnungsfaktor, ein Enzym, ein Hormon oder eine Hormonvariante; ein Rezeptor oder Teile davon; ein regulatorisches Protein; ein Strukturprotein; ein Reporter; oder ein Transportprotein.

## Revendications

1. Séquence d'acide nucléique qui comprend la séquence d'ADN représentée par SEQ ID NO : 48 et codant pour le polypeptide représenté par SEQ ID NO : 49, lequel polypeptide possède une activité d'activation de la transcription.

2. Construction d'acide nucléique comprenant la séquence d'acide nucléique de la revendication 1 liée de manière fonctionnelle à une ou plusieurs séquences de contrôle qui dirigent la production du polypeptide dans un hôte d'expression approprié.

3. Vecteur d'expression comprenant la construction d'acide nucléique de la revendication 2, un promoteur et des signaux d'arrêt de la transcription et de la traduction.

4. Cellule hôte non humaine comprenant le produit de construction d'acide nucléique selon la revendication 2 ou le vecteur d'expression selon la revendication 3.

5. Polypeptide qui est codé par la séquence d'ADN représentée par SEQ ID NO : 48.

6. Procédé de production du polypeptide selon la revendication 5 comprenant (a) la culture de la cellule hôte selon la revendication 4 dans des conditions favorables à la production du polypeptide ; et (b) la récupération du polypeptide.

7. Cellule hôte fongique utile pour la production d'un polypeptide, où la cellule :
a) est un mutant d'une cellule fongique parente où la cellule parente comprend une ou plusieurs séquences d'ADN codant pour une protéase dont la transcription est activée par un activateur de la transcription codé par une séquence d'acide nucléique selon la revendication 1 ; et
b) produit moins d'activateur de la transcription et de protéase(s) que la cellule parente lorsqu'elle est cultivée dans les mêmes conditions, et où la production réduite de l'activateur de la transcription est obtenue par modification ou inactivation d'une séquence d'acide nucléique codant pour l'activateur de la transcription représenté par SEQ ID NO : 49, ou par modification ou inactivation d'une séquence de contrôle nécessaire à l'expression du polypeptide activateur.

8. Cellule hôte selon la revendication 7, dans laquelle la séquence de contrôle est une séquence promoteur ou une partie fonctionnelle de celle-ci.

9. Cellule hôte fongique utile pour la production d'un polypeptide, où la cellule hôte est un mutant d'une cellule parente, où le mutant :
a) produit plus d'un activateur de la transcription codé par une séquence d'acide nucléique selon la revendication 1 par rapport à la cellule parente lorsqu'elle est cultivée dans les mêmes conditions, par introduction dans la cellule parente d'une ou plusieurs copies : (i) d'une séquence d'acide nucléique selon la revendication 1, (ii) de la construction d'acide nucléique selon la revendication 2, ou (iii) du vecteur d'expression selon la revendication 3, et
b) comprend une séquence d'ADN codant pour le polypeptide dont la transcription est activée par l'activateur de la transcription.

10. Cellule hôte selon la revendication 9, dans laquelle la séquence d'acide nucléique codant pour l'activateur de la transcription est liée de manière fonctionnelle à un promoteur qui est plus fort que le promoteur correspondant de la cellule parente.

11. Cellule hôte selon la revendication 10, dans laquelle le promoteur médie l'expression d'un gène codant pour une protéase extracellulaire.

12. Cellule hôte fongique utile pour la production d'un polypeptide, où la cellule est un mutant d'une cellule parente, où le mutant comprend :
a) une modification ou une inactivation d'un activateur de la transcription qui est codé dans une séquence d'acide nucléique native selon la revendication 1, ou par modification ou inactivation d'une séquence de contrôle nécessaire à l'expression du polypeptide activateur, et
b) (i) un promoteur inductible lié de manière fonctionnelle à une séquence d'acide nucléique selon la revendication 1, et (ii) une séquence promoteur qui peut être activée par l'activateur de la transcription codé par la séquence d'acide nucléique selon la revendication 1, liée de manière fonctionnelle à une séquence d'acide nucléique codant pour le polypeptide, où (i) et (ii) peuvent être introduits simultanément ou séquentiellement.

13. Cellule hôte selon la revendication 12, dans laquelle la séquence promoteur, ou une partie fonctionnelle de celle-ci, provient d'un gène de protéase.

14. Procédé de production d'un polypeptide, comprenant :
(a) la culture de la cellule hôte selon l'une quelconque des revendications 7 à 13, où la cellule hôte abrite un gène codant pour le polypeptide, dans un milieu nutritif approprié pour la production du polypeptide ; et
(b) la récupération du polypeptide à partir du milieu nutritif de la cellule mutante.

15. Procédé selon la revendication 14, dans lequel le polypeptide est natif à la cellule parente ou dans lequel le polypeptide est hétérologue à la cellule parente.

16. Procédé selon la revendication 14 ou 15, dans lequel le polypeptide est un anticorps ou des portions de celui-ci, un antigène, un facteur de la coagulation, une enzyme, une hormone ou un variant hormonal ; un récepteur ou des portions de celui-ci ; une protéine régulatrice ; une protéine structurale ; un rapporteur ; ou une protéine de transport.
